(19) Europäisches Patentamt
European Patent Office
Office européen des brevets

(11) EP 4 779 644 A2

(12) **EUROPEAN PATENT APPLICATION**

(43) Date of publication:
**22.07.2026 Bulletin 2026/30**

(21) Application number: **26178519.0**

(22) Date of filing: **28.04.2021**

(51) International Patent Classification (IPC):
***G16C 20/10*** *(2019.01)*

(52) Cooperative Patent Classification (CPC):
**G16C 20/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2020 GB 202006243**

(62) Document number(s) of the earlier application(s) in accordance with Art. 76 EPC:
**21724742.8 / 4 143 832**

(71) Applicant: **Chemify Limited**
**Glasgow G12 8QQ (GB)**

(72) Inventors:
- **CRONIN, Leroy**
  **Glasgow, G12 8QQ (GB)**
- **MEHR, Hessam**
  **Glasgow, G12 8QQ (GB)**
- **CRAVEN, Matthew**
  **Glasgow, G12 8QQ (GB)**
- **LEONOV, Artem**
  **Glasgow, G12 8QQ (GB)**

(74) Representative: **Mewburn Ellis LLP**
**Aurora Building**
**Counterslip**
**Bristol BS1 6BX (GB)**

Remarks:
This application was filed on 13-05-2026 as a divisional application to the application mentioned under INID code 62.

(54) **AUTOMATED CHEMICAL SYNTHESIS PLATFORM**

(57) The invention provides a method for controlling an automated chemistry platform using synthetic procedures written in natural language. The method uses natural language processing (NLP) techniques to interpret a synthetic procedure written in natural scientific language and extracts a machine-readable instruction set comprising the distinct operations necessary for carrying out the synthesis on an automated chemical synthesis platform. The method optionall also includes the step of executing the machine-readable instruction set on an automated chemical synthesis platform.

EP 4 779 644 A2

## Description

### *Related Application*

The present case claims priority to, and the benefit of, GB 2006243.6 filed on 28 April 2020 (28.04.2020), the contents of which are hereby incorporated by reference in their entirety.

### *Field of the Invention*

The present invention relates to a method for controlling an automated chemical synthesis platform, and a controller for an automated chemical synthesis platform.

### *Background*

The automation of chemical synthesis is an ongoing technological challenge. Typically, the successful automation of synthetic procedures is limited to a few well-defined areas, such as polypeptide and oligonucleotide synthesis. These areas are characterised by the ability to synthesis the desired target molecule by the successive iteration of a small palette of similar chemical reactions (e.g. amide coupling, phosphoramidite coupling), rendering them amenable to automation. Alternatively, the large-scale industrial synthesis of certain commodity chemicals may involve the automation of a diverse assortment of chemical reaction types. However, the large scale, commodity nature of these processes justifies the time and effort required to provide bespoke automation solutions for each synthetic step.

In contrast, the laboratory-scale synthesis of complex molecules is still a predominantly manual process, as the small-scale nature of each synthesis rarely justifies the extra effort required to automate each, often unique, reaction step. Indeed, even valuable fine chemicals such as pharmaceuticals, which are often produced in scales of up to 100 kg, are still typically produced in batch processes characterized by large number of manual handling operations.

The present inventors have previously disclosed an automated chemistry platform comprising the hardware necessary to perform a diverse range of synthetic reactions (*Steiner*). Dubbed the *Chemputer,* the device is able to automate batch-type synthetic procedures on a laboratory scale, and the *Chemputer* was used to synthesise pharmaceutical compounds such as diphenylhydramine hydrochloride, rufinamide and sildenafil without any human intervention.

However, though the *Chemputer* is able to automate such procedures, it is limited to executing a set of specialised low-level hardware instructions. Consequently, automation involved laborious and error-prone manual translation of a synthetic procedure into the required low-level instruction set. Quite simply, automation just displaced effort from manual labour to programming. Moreover, the instruction sets required to operate automated chemical synthesis platforms such as the *Chemputer* are often platform-specific, with no obvious semantic link between platforms from different vendors. Thus, a synthesis automated on one platform cannot be readily transferred to another platform. Operating each system requires both the relevant programming expertise and a detailed knowledge of the individual system's robotic operations. This knowledge cannot be readily transferred between platforms.

Finally, even though the last two hundred years of chemical research has yielded the discovery and synthesis of over 100 million different chemical species, the procedures describing the synthesis of the vast majority of these compounds are not in a machine-readable format. Typically, such synthetic procedures are only made available in prose, and this may be incomplete and ambiguous. Simple points are often omitted, as they are deemed obvious to a skilled chemist. As such, expert interpretation is required to fill in these gaps before a procedure can be translated into a machine-readable instruction set. This broken link has prevented the automation of synthesis: the vast repertoire of synthetic knowledge in the scientific literature cannot be directly executed by robots today.

The present invention has been devised in light of the above considerations.

### *Summary of the Invention*

At its most general, the present invention relates to a method for controlling an automated chemistry platform using synthetic procedures written in natural language. The method uses natural language processing (NLP) techniques to interpret a synthetic procedure written in natural scientific language and extract a machine-readable instruction set comprising the distinct operations necessary for carrying out the synthesis on an automated chemical synthesis platform.

Importantly, the method also establishes the physical equipment necessary to perform the synthesis. That is, the system can compile both a list of operations (e.g. add, stir, heat, cool, filter mix) and a list of the equipment (vessels with heating and cooling ability) and hardware requirements (e.g. temperature ranges) necessary to perform a given synthesis. Therefore, the method can control a synthesis using any automated chemical synthesis platform meeting the hardware requirements.

As such, the method makes the extensive natural language chemical literature directly executable on an

automated chemical synthesis platform. A chemist with no knowledge of programming can use an automated platform to synthesis a compound based only on natural language synthetic procedures.

In a first aspect of the invention, there is provided a method for controlling an automated chemical synthesis platform, the method comprising:

(a) providing a synthetic procedure in natural language;
(b) tagging the synthetic procedure with *Subject, Action* and *Modifier* tags;
(c) producing a machine-readable instruction set from the tagged synthetic procedure;
(d) generating a hardware index from the instruction set; and
(e) checking the hardware capability of the automated chemical synthesis platform against the hardware index, and optionally
(f) executing the instruction set on an automated chemical synthesis platform meeting the hardware capabilities.

Tagging the synthetic procedure may comprise using pattern matching to apply the *Action* and *Modifier* tags.

*A Subject* tag contains the identity of a chemical compound, and optionally comprises the absolute or relative amount of a compound for use in the synthetic procedure. A chemical compound may be indicated using a *Reagent* tag.

Tagging the synthetic procedure may comprise generating a list of candidate reagent names from the synthetic procedure, checking the list of candidate reagent names against a list of commonplace reagent names, and applying a *Regent* tag to candidate reagent names which match a commonplace reagent name. The *Subject* tag is assembled from text comprising the *Reagent* tag using pattern matching. Checking the list of candidate reagent names against a list of commonplace reagent names provides a rapid method for identifying chemical reagents.

Tagging the synthetic procedure may comprise generating a list of candidate reagent names from the synthetic procedures, extracting word fragments of 2 to 4 characters from the candidate reagent names, calculating the probability that the word fragments are part of a chemical compound name or part of normal text, and applying a *Reagent* tag to candidate reagent names which meet a probability threshold. The *Subject* tag is assembled from text comprising the *Reagent* tag using pattern matching. The probability may be calculated using a Naïve Bayes classifier. Comparing 2 to 4 character word fragments provides an exhaustive method for identifying chemical reagents (a method with a low rate of false negatives).

Extracting the instruction set may comprise:

(a) combining an *Action* tag with a *Modifier* tag to give a pair, typically based on their relative location in a sentence; and
(b) combining the *Action* and *Modifier* pairs with a tagged *Subject* to give an operation, typically using pattern recognition.

The instruction set is in machine-readable form. Typically, the instruction set is in a mark-up language, such as a descriptive mark-up language, for example *XML.* Preferably, the instruction set is in a mark-up language adapted for chemical synthesis, such as *XDL. XDL* allows chemical procedures to be described in a general, platform-independent manner. A synthesis stored as an XDL file can be interpreted and executed on many automated chemical synthesis platforms.

The method may comprise outputting the instruction set in natural language. This allows the instruction set to be compared against the native synthetic procedure. A discrepancy arising from, for example, a mistranslation or missing information, can be corrected. This increases the fidelity of the method. This step is typically undertaken as part of step (c).

The hardware index may include a description of the physical hardware required to perform the synthesis and a description of the necessary connections between the hardware.

The hardware index may be in graph format, such as GraphML.

The automated chemical synthesis platform may be a *Chemputer.*

In a second aspect, there is provided a controller for an automated chemical synthesis platform, wherein the controller is configured to:

(a) tag a synthetic procedure in natural language with *Subject, Action* and *Modifier* tags;
(b) produce a machine-readable instruction set from the tagged synthetic procedure;
(c) generate a hardware index from the instruction set;
(d) check the hardware capabilities of the automated chemical synthesis platform against the hardware index; and
(e) execute the instruction set on an automated chemical synthesis platform if it meets the required hardware capabilities.

These and other aspects and embodiments of the invention are described in further detail below.

***Summary of the Figures***

The present invention is described with reference to the figures listed below.

**Figure 1** provides an overview of a method according to an embodiment of the invention. The method takes synthetic procedures described using natural language and converts them to a hardware-independent instruction set (e.g. XDL), which can be optionally represented in natural language for human inspection or verification. A hardware index (e.g. inferred graph) can be automatically generated from the instruction set. This can be compared with the corresponding template of the user's automated chemical synthesis platform. A compilation step then combines the instruction set with the hardware index to produce a hardware-specific executable suitable for immediate execution on the target platform

**Figure 2** a) Shows an overview of a process by which an input text in natural language is converted to *XDL.* First, the text is hierarchically tagged by pattern matching. Pattern matching is then used again to extract all actions from the labelled text with their accompanying subjects and modifiers. Finally, the extracted actions are converted to *XDL.* The example text here contains only one action, but the system can handle multiple actions in one sentence. b) Demonstrates error detection in the input text. A user can rectify translation errors simply using the natural language output from the system.

**Figure 3** describes chemical schemes and the corresponding abstract chemical processes for the synthesis of (a) lidocaine; (b) DMP and its use in oxidising menthol to menthone; and (c) *AlkylFluor,* along with the particular operations that need to be performed by an automated chemical synthesis platform.

**Figure 4** provides an overview of the method by which an input text in natural language is converted to an instruction set (here, in *XDL*). Unused phrases output from the method in the natural language representation of the instruction set can be used to error correct the original text, in this case fix the spelling mistake "healted". A hardware index (here, a graph) is then generated from the instruction set, which is compiled with the instruction set into the low-level code for the platform controller (here, a ChemEXE file). The synthesis can be simulated in order to spot any potential sources of error before physical execution on the automated chemical synthesis platform.

**Figure 5** is a key showing the different device types encountered in the device layout diagrams in Figures 6 to 11.

**Figure 6** shows the hardware layout used for the synthesis of lidocaine.

**Figure 7** shows the hardware layout used for the synthesis of DMP and oxidation of menthol using DMP.

**Figure 8** shows the hardware layout used for the synthesis of AlkylFluor (steps 1-4).

**Figure 9** shows the hardware layout used for the synthesis of AlkylFluor (step 5).

**Figure 10** shows the hardware layout used for the synthesis of a $W_{19}Mn_2Se_2$ polyoxometalate cluster on a *Chemputer.*

**Figure 11** shows the hardware layout for the synthesis of a $W_{19}Mn_2Se_2$ polyoxometalate cluster on a *Clusterbot.*

***Detailed Description* of *the Invention***

The present invention relates to a method for controlling an automated chemical synthesis platform, and a controller for an automated chemical synthesis platform. The method may be a computer-implemented method.

The methods of the present case address the issues with the work described in Steiner *et al.,* which required bespoke programming for a bespoke system, and provides no general approach to translating natural language instructions into executable processes.

An overview of an embodiment of the method is provided in Figure 1. The method takes synthetic procedures in natural language and converts them to a hardware-independent machine-readable instruction set (e.g. XDL), which can be optionally represented in natural language for human inspection or verification. A hardware index (inferred graph) can be automatically generated from the instruction set. This index is compared with the corresponding template of the user's automated chemical synthesis platform. A compilation step then combines the instruction set with the hardware index to produce the hardware-specific instructions suitable for execution on the target platform. A similar overview of an

embodiment is shown in Figure 4, including an additional simulation step which can act as an error-checking process to spot any potential sources of error before a physical run.

An overview of the process by which text is converted to *XDL* is shown in Figure 2a. First, the text is tagged by pattern matching. Pattern matching is again used to extract all actions from the labelled text with their accompanying subjects and modifiers. Finally, the extracted actions are converted to *XDL.* The example text here contains only one action, but the system can handle multiple actions in a single synthetic procedure.

As is described in further detail below, and as is apparent from the worked examples, the methods and controller of the invention allow any synthetic procedure written in natural language to be converted into a machine readable instruction and associated hardware index and so executed on any suitable automated chemical synthesis platform.

*Automated Chemical Synthesis Platform*

An automated chemical synthesis platform is a robot capable of executing a chemical synthesis with limited human intervention. Automated synthesis platforms are known.

The capabilities of automated chemical synthesis platforms vary widely. Typically, automated chemical synthesis platforms are specifically adapted to perform a select number of operations. For example, automated chemical synthesis platforms may comprise a series or array of reaction vessels along with a means for adding reagents to the reaction vessels.

Different means for adding reagents to the reaction vessels are known. For example, a robotic pipetting arm may be used to aspirate a certain volume of fluid from one vessel and dispense this into another vessel. Alternatively, a pump may be used to pump fluids from one vessel into another vessel.

Typically, the reaction vessels in an automated chemical synthesis platform are equipped for stirring. Optionally, they may be equipped for heating and/or cooling.

A *Clusterbot* is an example of a simple automated synthesis platform (*Steiner*). The *Clusterbot* can perform operations such as addition of liquid reagents and stirring of a mixture within a reaction vessel. Such automated synthesis platforms can be used in the methods described herein.

Alternative automated synthesis platforms can perform a wider number of operations. Such platforms may be termed general automated synthesis platforms.

Additional operations that may be performed by general automated chemical synthesis platforms include separations such as filtrations, liquid-liquid extraction and chromatographic separation. Operations such as evaporation to dryness or reflux under controlled pressure may be performed. General automated chemical synthesis platforms may be equipped for handling sensitive reagents using inert gas blanketing.

General automated chemical synthesis platforms may also be capable of analytical measurement of reaction products. Example analytical techniques include conductivity sensors (for pH measurement), cameras (for colour change measurement), UV-VIS spectroscopy, IR spectroscopy, NMR spectroscopy and mass spectroscopy. LCMS or GCMS may be used. The inclusion of analytical equipment allows the platform to carry out live reaction monitoring. This allows the system to continue a reaction until an appropriate end-pint has been reached (e.g. heating a reaction until a certain peak disappears in the liquid chromatograph trace of the reaction mixture).

A *Chemputer* is an example of an automated chemical synthesis platform that can perform a wide number of operations (*Steiner*). The *Chemputer* is a modular platform comprising different modules tailored for performing different operations. The *Chemputer* comprises a fluidic manifold (or "backbone") consisting of a series of pumps coupled with multi-directional valves which can transfer material to different modules connected to the manifold. The modules include, for example, a reactor module, a liquid-liquid extraction module, a solvent evaporation module, a filtration module and a column chromatography module. The manifold can be elongated to account for additional modules. The *Chemputer* may monitor reactions using conductivity sensors and can be equipped to automatically sample reactions for analysis by methods including UV-VIS spectroscopy, IR spectroscopy, NMR spectroscopy or mass spectrometry.

An example *Chemputer* platform that is compatible with the control method of the invention may use the following software release versions:

| Component | Release Version |
|---|---|
| XDL | v0.4.2 |
| Chemputer XDL | v0.1.1 |
| Chempiler | v2.0.6 |
| ChemputerAPI | v2.0 |
| SerialLabware | v1.1.1 |

*Synthetic Procedure*

The method comprises providing a synthetic procedure in natural language. This is step (a). A synthetic procedure is a description of a method of making a chemical product. A synthetic procedure typically comprises the different experimental operations or steps required to produce the product. It may comprise information on the different reagents (e.g. starting materials and reactants), solvents and catalysts, and the quantities required, to produce the product. It may comprise information on any specialist equipment needed. However, it is typical for a synthetic procedure to omit certain specific information that may be deemed obvious to a skilled chemist. Typically, information on the necessary equipment is omitted. Similarly, information on work-up (e.g. quenching of the reaction mixture), flushing (e.g. with inert gases), washing, extraction and purification steps is also omitted.

A synthetic procedure is typically found in an academic journal article in the chemical, biological or materials sciences. It may be located either in the main text of the article, or in the associated supplementary information. A synthetic procedure may also be found in other academic texts, such as a review article, letter or book. It may be found in a specialised synthetic text book or a laboratory standard operating procedure. Dedicated web repositories for synthetic procedures are also known (e.g. http://www.orgsyn.org). They may also be found in teaching materials, such as a general textbook or a laboratory procedure. The procedure may also be set out in a thesis, or a company report.

Synthetic procedures are typically in natural language. That is, in free, unstructured text. For example, in prose. These natural language synthetic procedures are suitable for use in the present invention.

The method may comprises an optional pre-processing step. The pre-processing stage can be used to simplify and shorten the natural language synthetic procedure to make the subsequent processing stages quicker. Several pre-proceedings steps may be included. The pre-processing steps are performed automatically.

The pre-processing step may comprise performing optical character recognition (OCR) on the document displaying the natural language synthetic procedure. OCR converts an image of text, such as scanned text or a photograph of text, into machine-encoded text. Thus, including OCR enables the method to use scanned or photographed synthetic procedures from physical (hard-copy) articles or handwritten notebooks.

The pre-processing step may comprise removing HTML from the synthetic procedure. HTML code is typically included in synthetic procedures contained in an electronic database. For example, synthetic procedures in the Reaxys database typically contains HTML code. It is not necessary to understand the HTML in order to extract an instruction set from the synthetic procedure.

The pre-processing step may comprise removing formatting markings from the synthetic procedure. For example, the pre-processing steps may comprise removing formatting marks indicating italicisation, emboldening and underling.

The pre-processing step may comprise removing non-printing characters from the synthetic procedure. For example, the pre-processing step may comprise removing tabs and paragraph markers (carriage returns). Non-breaking characters may be replaced with standard counterparts (e.g. replacement of a non-breaking space with a space).

The pre-processing step may comprise removing analytical data from the synthetic procedure. Analytical data includes, for example, NMR, IR, melting point and mass spectrometry data. This analytical data can be useful for verifying the identity of chemical compounds, for example, the final product. This analytical data may be termed characterisation data. Characterisation data is typically found at the end of a synthetic procedure, or at the end of each step within a synthetic procedure. However, characterisation data is not necessarily required for the extraction of the instruction set. Removing the characterisation data in a pre-processing step reduces the text, speeding up subsequent steps.

Certain analytical data may be useful for performing the synthetic procedure. For instance, analytical data defining the end-point of a reaction or synthetic operation. For example, where a synthetic procedure requires a synthetic operation to be performed until a given colour change is observed, or until a certain peak within the chromatograph of the reaction mixture disappears. This analytical data may also include the appearance or disappearance of specific peaks within an NMR or IR spectrum of the reaction mixture. This analytical data may be termed reaction-monitoring data. Reaction monitoring data is typically found embedded within the text of the natural language synthetic procedure. Optionally, this analytical data may be retained in the synthetic procedure.

The pre-processing may comprise normalising the text. This transforms the text into a consistent format. For example, normalising the text may comprises inserting missing spaces after full stops or before certain units, such as "°C".

The pre-processing step may comprise translating the text, for example, translating the text from German, French, Chinese, Japanese, Korean or Russian into English. The method may use a single language, preferably English, for standardisation within the system.

Finally, the pre-processing step may include making specific replacements to standardize the appearance of certain phrases. For example, words denoting numbers may be replaced with their respective numerals (e.g. replacement of "ninety nine" with "99"). Full stops that denote abbreviations may be removed (e.g. replacement of "min." in the phrase "5 min." with "min" to give "5 min"). The different characters used to represent the degree symbol may be replaced with the standard symbol (e.g. replacement of the letter o in superscript "$^{O}$" or "$^{O}$" or the ordinal indicator "º" with the degree symbol

"°").

*Tagging*

Tagging is the automated process of assigning grammatical roles to phrases in the natural language text of the synthetic procedure. This is step (b). The tagging step creates labelled (tagged) entities from the text. That is, attaching a marker (tag) to fragments of the text to provide additional information, such as an indication of its grammatical role or to identify it as belonging to a certain class of words.

Tagging of natural language scientific data is known. For example, *ChemicalTagger* is a tagger which uses natural language processing techniques to extract structured scientific data from synthetic procedures (*Hawizy*). However, *ChemicalTagger* is designed for the extraction of information (e.g. physical properties such as melting point), in order to allow that information to be easily searched or grouped. *ChemicalTagger* is not capable of outputting a machine-readable instruction set for the purposes of controlling an automated chemistry platform.

In the present method, the tagging step identifies and tags three primary classes of information within the text that are designated *Subject, Action,* and *Modifier.*

A *Subject* tag contains the identity of a chemical compound and the relative or absolute amount of that compound. That is, a *Subject* contains a chemical name and a quantity. For example, "triethylamine (5 mL)". Thus, typically, a *Subject* tag is linked to nouns or noun phrases in the natural language synthetic procedure.

An *Action* tag is linked to an operation in the natural language synthetic procedure. For example, "add", "heat", "cool", "filter", "mix". Typically, an *Action* tag is linked to a verb in the natural language synthetic procedure. The verb may be found in any tense, and in both passive and active language.

A *Modifier* tag is linked to an adaption of an *Action.* Typical *Modifiers* are concerned with time, temperature and pressure, amongst others. For example the *Action* "add" may be adapted by the *Modifier* "dropwise over 30 minutes" or the *Action* "cool" may be adapted by the *Modifier* "to -10 °C". Typically, *Modifiers* liked to adverbs in the natural language synthetic procedure.

The tags are applied automatically. That is, the method comprises automatically tagging the synthetic procedure with *Subject, Action* and *Modifier* tags. A suitably programmed controller is provided to undertake the tagging operation.

The tagging step comprises recursively parsing the text of the synthetic procedure to create labelled entities from matched patterns. The labelled entities are hierarchically tagged (see Figure 2).

The tagging step uses pattern matching to apply all low level tags except for the *Reagent* tag. Patterns can be set (defined), or can be programmatically generated from smaller defined patterns. At this stage, the pattern matching is used to analyse small text fragments. Example of patterns are shown in Table 1.

**Table 1:** Examples of patterns matched by SynthReader

| **Pattern** | **Tag** | **Example phrase matched by pattern** |
|---|---|---|
| *Number,* "mL" | *Volume* | 50 mL |
| *Number,* "M" | *Conc* | 1M |
| **Pattern** | **Tag** | **Example phrase matched by pattern** |
| *Number,* "minutes" | *Time* | 30 minutes |
| *Number,* "°C" | *Temp* | -10 °C |
| *Number,* "bar" | *Pressure* | 10 mbar |
| "a", *Conc,* "aqueous", "solution", "of", *Reagent,* "(", *Volume,* ")" | *Solution* | A 1 M aqueous solution of NaOH (50 mL) |
| "over", "a", "period", "of", *Time* | *TimeModifier* | over a period of 30 minutes |
| "to", *Temp,* "or", "below" | *TempModifier* | to -10 °C or below |
| "evaporated" | *Action* | evaporated |
| "added" | *Action* | added |
| "heated" | *Action* | heated |
| "cooled" | *Action* | cooled |
| "fileted" | *Action* | filtered |

Where appropriate, different units may be recognised during the tagging stage. For example, the *Volume* tag may be applied to a fragment comprising a number followed by any of the units *mL* and *L*. The *Time* tag may be applied to a fragment comprising a number followed by any of the units *s*, *sec, secs, second, seconds, m min mins, minute, minutes, h hr, hrs, hours,* and *hours.* The *Temp* tag may be applied to a fragment comprising a number followed by the units *°C* or *K,* or it may be applied to specific phrases such as *"room temperature". The Pressure* tag may be applied to a fragment comprising a number followed by the any of the units *bar, mbar, Pa,* and *kPa.*

The *Reagent* tag may be applied to any chemical entity, including starting materials, reagents, solvents, catalysts and washing solutions.

The tagging step may apply the *Reagent* tag by first generating a list of candidate chemical reagent names. That is, a list of phrases which may represent the names of chemical reagents.

The list of candidate chemical reagents may be generated by generating a list of all possible phrases in the text and filtering the list of all possible phrases. The filtering may comprise discarding phrases containing certain words that are unlikely to be in a reagent name, discarding phrases that contain certain words at the end of a phrase, and/or disregarding phrases below a certain minimum character threshold.

The list of candidate phrases may be checked against a list of commonplace chemical or biological terms or reagent names. Commonplace reagent names may be systematic names (e.g. IUPAC names) or common (e.g. trivial) names. Lists of commonplace chemical names are known. A bespoke list of commonplace reagent names has been compiled and is available at:
https://gitlab.com/croningroup/chemputer/chemdata/-/blob/master/chemdata/synonyms.py [accessed 30 March 2020].

If a match is found, the *Reagent* tag is applied to the phrase.

Optionally, if a match is found, the method comprises checking the word before and/or after the candidate phrase to see if the compound phrase (candidate phrase + preceding or trailing word) is found in the list of common reagent names. For example, if 'acetic acid' matches, but the full sentence reads '...in glacial acetic acid.', then 'glacial acetic acid' would be the reagent name tagged as the word 'glacial' is recognised as a reagent name preceding word. This reduces errors.

The list of candidate phrases may also be checked using a probabilistic classifier. For example, two-, three- and/or four-letter fragments may be extracted from the candidate phrase and the probability of these fragments appearing in a reagent name may be calculated.

The probability may be calculated by comparing the likelihood of the fragment appearing in a chemical reagent database to the likelihood of the fragment appearing in a natural language database. Chemical reagent databases include, for example, the Reaxys database. Natural language databases include, for example, the Brown corpus.

The probability of the fragment appearing in a reagent name may be calculated using a Naïve Bayes classifier. For example, using equation (1), where *R* means phrase is a reagent name, and *N R* means phrase is not a reagent name.

$$P(R|\text{phrase}) = \frac{\sum_{i=0}^{n} \frac{P(\text{feature}_i|R)P(R)}{P(\text{feature}_i|R)P(R)+P(\text{feature}_i|NR)P(NR)}}{n} \quad (1)$$

If the probability reaches a certain threshold, the *Reagent* tag is applied to the phrase.

After *Reagent* tags have been applied, *Subject* tags are assembled from *Reagent* tags. Pattern matching is used to assemble the *Subject* tags from the *Reagent* tags. At this stage, pattern matching is used to analyse larger text fragments, such a whole clauses or sentences. Text with the *Reagent* tag may be labelled at with a *Subject* if the correct pattern is found. Optionally, text with the *Regent* tag may be labelled as a *Modifier* depending on the pattern. Examples of pattern matching for applying the *Subject* tags are given in Table 2.

**Table 2:** Examples of pattern matching for applying the *Subject* tag

| Pattern | Example phrase matched by pattern | Subject | Action(s) | Modifiers (part of action in pattern) |
|---|---|---|---|---|
| Subject, Auxiliary verb, Action | Trimethylamine (5 mL) was added dropwise | Trimethylamine (5 mL) | added | dropwise |
| Subject, Auxiliary verb, Action | The product was extracted with ethyl acetate (20 mL). | The product | extracted | with ethyl acetate (20 mL) |
| Subject, Auxiliary verb, Action, 'and', Action | The mixture was filtered and dried for 2 hrs | The mixture | filtered, dried | for 2 hrs |

*Instruction Set*

The method comprises the step of producing a machine-readable instruction set from the tagged synthetic procedure. This is step (c). An instruction set comprises a list of the individual operations needed to perform a synthesis. The instruction set can be interpreted by an automated chemical synthesis platform, and the platform can act upon that instruction set.

The instruction set may be in any suitable machine readable format. Typically, the instruction set is in a mark-up language, such as a descriptive mark-up language, for example *XML.* The preferred format for the instruction set is a mark-up language adapted for chemical synthesis, such as *XDL. XDL* is described in (*Steiner*).

The production of the machine-readable instruction set from the tagged synthetic procedure may comprise an interpretation step and a conversion step.

*Interpretation*

The interpretation step takes the tagged synthetic procedure and extracts a list of *Actions,* each action accompanied by a list of *Modifiers.* The interpretation step combines *Modifiers* with *Actions.* Typically, *Modifiers* are combined with *Actions* based on their relative position (proximity) in the natural language synthetic procedure.

The interpretation step may comprise removing superfluous words from the text. This reduces the length of the text and improves the efficiency of the procedure. Superfluous words are typically unused adverbs or adjectives. Examples of superfluous words commonly found in synthetic procedures include "then", "subsequent", "again", "first", "successively", "along", "additional", "sequentially", "further" and "next".

The interpretation step may comprise resolving anaphors. An anaphor is a pointer to a word or phrase appearing earlier in the text. An example of an anaphor is the word "this" in the phrase "the mixture was heated to this temperature". Anaphors are resolved by replacing the anaphor with the actual word or phrase that is referred to. Typically, the anaphor can be resolved by searching through the preceding text until text having the correct tag is found. For example, the text preceding the phrase "this temperature" is searched for a word having the *Temperature* tag.

The interpretation step comprises combining the *Action* and *Modifier* pairs with the *Subjects* to generate an operation list. Typically, pattern matching is used to construct the operation list from the *Action* and *Modifier* pairs and *Subjects.* In the interpretation stage, pattern matching is used to analyse entire clauses or sentences. Examples of pattern matching in interpretation are given in Table 3.

**Table 3:** Pattern matching during the operation phase

| Pattern | Example phrase matched by pattern |
|---|---|
| *Subject, AuxiliaryVerb, Action,* "and", *Action* | Ethanol (50 mL) was added and heated to 30°C |
| *Subject, AuxiliaryVerb, Action, Action,* "and", *Action* | The product was filtered, washed with water (3 x 50 mL) and dried for 6 h. |
| *Subject, AuxiliarVerb, Action, Action,* "followed", "by", *Action* | The organic phase was separated, dried over Celite, followed by rotary evaporation (40 °C, 50 mmHg). |

*Conversion*

The conversion step comprises converting the operation list into a machine-readable instruction set. An instruction set is a machine-readable list of the operations that need to be carried out in order to perform a synthesis.

Typically, the instruction set comprises a standardised list of operations. Each operation is accompanied by a standardised list of attributes.

The instruction set may be in any format suitable for machine-reading. Typically, the instruction set is in a mark-up language, such as a descriptive mark-up language, for example *XML.* The preferred format for the instruction set is a mark-up language adapted for chemical synthesis, such as *XDL. XDL* is described in (*Steiner*).

Conversion of the operation list into the instruction set can include action sanitisation. Action sanitisation checks that an individual operation in the operation list has all the required attributes necessary for the operation to be performed by an automated chemical synthesis platform. Typically, action sanitisation comprises checking each operation in the operation list against a list of known operations and their associated attributes.

*Natural Language Output*

Natural language is extremely flexible, and written synthetic procedures may be incomplete or ambiguous. A

synthetic procedure in natural language may contain transcription errors such as grammatical or spelling errors. Therefore, the tagging process may be incomplete and information in the natural language synthetic procedure may be missed. In order to check the fidelity of the extracted instruction set, the present invention provides an optional natural language text output. That is, the method comprises outputting the instruction set in natural language. In this way, the natural language output can be compared to the underlying natural language synthetic procedure and any mistranslation or missing information can be detected.

A discrepancy between the natural language synthetic procedure and the natural language output can be corrected. This correction may comprise editing the natural language synthetic procedure (input), for example, to correct previously undetected spelling errors or typos, or to include additional missing information.

The comparison and/or correction may be performed by the user. Thus, users with no programming experience can interactively resolve ambiguities in the original text or amend any missing or implicit process variables, as shown in Figure 1b.

The natural language output can be in any suitable format, for example prose. Typically, the natural language output is a list of steps. This improves error checking, as it is easy to locate the source of any errors. An example natural language output produced by an embodiment of the invention is shown in the synthesis Lidocaine, below (Example 2).

The present invention also comprises additional natural language outputs comprising specific information. For example, the invention provides an output table indicating the amount (e.g. volume, mass or molar quantity) of reagents that will be consumed during the process. Optionally, the invention provides an output indicating an estimated duration of the whole procedure.

*Hardware Index*

A hardware index is generated from the instruction set. This is step (d).

The hardware index contains a description of the physical hardware required to perform the synthesis, including a description of the necessary physical and fluidic connections between the hardware. The description of the physical hardware may be a description of the separate hardware modules (units) that are required. Example modules include modules for filtration, liquid-liquid extraction, chromatographic separation, evaporation, and heating under reflux at controlled pressure, as well as reaction vessels.

Typically, the hardware index also includes a description of any necessary parameters the hardware must achieve in order to perform the synthesis. For example, if the synthesis requires heating to a given temperatures (e.g. 70 °C), the hardware index includes the information that the reaction vessel must be capable of heating to that temperature (e.g. 70 °C). Typical parameters included in the hardware index include volume (the capacity to hold a certain quantity of material), temperature (both heating and cooling) and pressure (raised or lowered).

Optionally, the hardware index also includes information necessary to safely perform the synthesis. For example, if the synthesis requires the use of fluorinating agents (e.g. AlkylFluor) or other fluorine-containing compounds, the hardware index includes the information that the reaction vessel must be resistant to fluorinating compounds (e.g. be a polymer and not glass). Typical safety information included in the hardware index includes fluorine reactivity, solvent reactivity (avoidance of certain polymers) and reagent compatibility (cross-reactivity).

Optionally, the hardware index includes a list of the physical analytical hardware useful for performing the synthesis, including a description of the necessary physical and fluidic connections to the analytical hardware. The analytical hardware may be separate analytical hardware modules (units). Example analytical modules include modules for pH measurement (e.g. conductivity sensors), colour change measurement (e.g. cameras), UV-VIS spectroscopy, IR spectroscopy, NMR spectroscopy, mass spectroscopy, liquid chromatography and gas chromatography.

Analytical hardware may be used for reaction monitoring, for example, in the case where reaction monitoring data is include in the synthetic procedure (see above). Alternatively, this analytical hardware may be used for characterisation of the final product and confirmation that the synthetic procedure was successful.

The hardware index typically also includes a list of the necessary reagent required as inputs into the synthesis. This may be as a list of the necessary reagent flasks and their physical and fluidic connections into the system.

Optionally, the hardware index includes a list of the necessary solvents, including buffers, and cartridges for drying (e.g. $MgSO_4$), filtration (e.g. Celite®) and chromatography apparatus (e.g. silica solid phase).

Preferably, the physical hardware (modules) and their connections are represented as a graph. That is, the method comprises generating a hardware graph from the instruction set. Different graph formats are known, such as GraphML. GraphML is an open-standard, extensible mark-up language (XML)-based exchange format for graphs (http://graphml.graphdrawing.org [accessed 19 October 2018]). The use of a graph to describe the layout of an automated synthesis platform is described by Steiner (*Steiner*). The graph may be directed.

Typically, in the graph layout, valves are represented as nodes and the interconnections (flow channels) between the valves are represented as edges. Other physical hardware modules (e.g. modules for filtration, liquid-liquid extraction, chromatographic separation, evaporation, and heating under reflux at controlled pressure, as well as reaction vessels)

may be represented as nodes. Containers for storage of reagents, solvents and waste may also be represented as nodes.

Within the graph representation, each node type may be assigned properties consistent with its role. Typical rules for assigning the assigning the properties are set out below. These properties may include information on its type, address and other relevant technical information.

A syringe pump may be represented as a node and have one edge representing the single entry and exit port.

A multi-directional valve may be represented as a node and have one edge per "port" on the valve. Each port may have an assigned unique identifier. Optionally, one port may be dedicated for connection to a syringe pump (an incoming edge) and the remaining ports may correspond to outgoing edges. If two valves are connected to each other outlet port to outlet port, two edges must exist between them, where the "port" properties of each edge represent the port from which they originate.

A reaction flask may be represented as a node and have one edge. Typically, heating or cooling devices (jacketed vessel, hotplate stirrer) are associated with a reaction vessel, as a user typically only wants to stir or heat a reaction flask. This can be achieved by saving the heating or cooling device type and addresses as node properties. Other equipment may associated with the reaction flask as appropriate (e.g. equipment for shaking or ultrasound).

A filter, may be represented as a node and have two edges. The first edge may be an incoming edge representing the line in to the vessel (into the top of the filter) and the second edge representing the line out of the vessel (out of the bottom of the filter, the filtrate collection vessel).

A liquid-liquid separator, may be represented as a node and have two edges. The first edge may be an incoming edge representing the line in to the vessel (into the top of the separator) and the second edge representing the line out of the vessel (out of the bottom of the separator, to collect the lower phase). Typically, a stirrer (e.g. overhead stirrer) and a conductivity sensor (to distinguish each phase) are associated with a liquid-liquid separator.

A rotary evaporator, may be represented as a node and have two edges. The first edge may be an incoming edge representing the line in to the vessel (into the evaporation flask) and the second edge representing the line out of the vessel (out of the distillate flask). Information required for communication (serial ports etc.) within the rotary evaporator can be saved as node properties.

In general, other equipment is represented as a node and has a single edge, and no two nodes that are both not valves may be connected.

The hardware graph may be generated automatically from the instruction set. The hardware index may be generated from the instruction set using a template graph. In such cases, the method may comprise providing a template graph and populating the template graph using the instruction set.

The template graph may comprise a description of the components in the automated chemical synthesis platform, their connectivity and their associated operational parameter ranges (e.g. available heating and cooling ranges). The template graph may be provided by the user, or automatically by the automated chemical synthesis platform.

Populating the template graph comprises comparing the template graph to the instruction set and removing those parts of the template graph not used. For example, if the template graph includes a module (node) for phase separation but the instruction set does not involve a phase separation operation, then the node corresponding to the phase separation module is removed. Any associated properties are also removed (for example, a conductivity sensor associated with a liquid-liquid separator).

Populating the template graph also comprises assigning empty nodes as containers for storage of the reagents and solvents listed in the instruction set. A waste container is also typically populated on the template graph.

Population of the template graph produces the final hardware graph.

*Hardware Check*

The hardware index may be checked against the physical capabilities of the automated chemistry platform. This is step (e). This may include checking if the required modules are present, if and heating and/or chilling modules are capable of reaching the required temperatures, and if there are enough free positions to add the required reagent flasks, buffer flasks, and chromatography cartridges.

If the check finds the hardware requirements are incompatible with the automated chemistry platform, an error message is produced.

Importantly, the procedure (instruction set) is independent of the hardware requirements (e.g. graph). Therefore, the hardware requirements can be edited by the user if desired. Similarly, the hardware requirements can be checked against different configurations of the automated chemistry platform, or different automated chemistry platforms.

Alternatively, the procedure (instruction set) may be edited as required. Certain adaptions may be suggested automatically. For example, if the procedure is carried out at a certain scale, but the automated chemistry platform can only handle a smaller volume of material, then the method may suggest a suitably scaled-down version of the procedure.

*Execution*

Optionally, the machine-readable instruction set is executed on an automated chemical synthesis platform meeting the hardware capabilities. This is step (f).

The execution of the instruction set may comprise a compilation step.

The instruction set is platform-independent. It represents a sequence of abstract synthetic operations that can be executed on any platform which meets the hardware requirements. The instruction set can be complied into the relevant platform-level instructions.

Processes for compilation of higher level code into relent machine level code will vary, and are typically provided by the vendor of each automated chemical synthesis platform. Typically, the instruction set is broken down into the constituent steps and the physical hardware is mapped to the instruction set. Each step may be checked against a standard step in the instruction set of the automated chemical synthesis platform to check that all the expected properties are present.

Where the instruction set is in *XDL,* the compilation process is described in *Steiner.* The core of the compilation process is the breakdown of the level *XDL* steps into their constituent substeps and the mapping of the platform hardware to the abstract *XDL* hardware. The mapped *XDL* is then solidified in an *xdlexe* file. The *xdlexe* file contains a hash of the graph representing the platform for which the file was compiled, and the file will only execute using a platform controller linked to this graph. Other platform specific alterations can also be made at this stage.

Execution of the machine-readable instruction set on an automated chemical synthesis platform results in the production of the final chemical product described in the synthetic procedure.

The execution step involves carrying out the operations necessary to perform the synthetic procedure. These operations typically include the transfer of reagents and solvents from the appropriate reservoirs to an appropriate reaction flask. Typically, the reaction required stirring and either heating or cooling.

Optionally, additional synthetic processes such as filtration, liquid-liquid extraction, chromatographic separation and evaporation may be required. The execution step may comprise transfer of the relevant reaction mixture to hardware specialised for those processes, along with operation of that hardware.

Optionally, analytical processes such as pH measurement (e.g. conductivity sensors), colour change measurement (e.g. cameras), UV-VIS spectroscopy, IR spectroscopy, NMR spectroscopy, mass spectroscopy, liquid chromatography and gas chromatography may be required. These processes may be required for reaction monitoring (e.g. perform an operation until a given colour change is observed) or for characterisation of the final product. The execution step may comprise operation of the relevant hardware specialised for those processes, along with transfer of the reaction mixture to appropriate sampling equipment.

*Controller*

The invention also provides a controller for controlling an automated chemical synthesis platform using the method set out above. That is, the invention provides a controller for an automated chemical synthesis platform, wherein the controller is configured to:

(a) tag a synthetic procedure in natural language with *Subject, Action* and *Modifier* tags;
(b) produce a machine-readable instruction set from the tagged synthetic procedure; and
(c) generate a hardware index from the instruction set;
(d) check the hardware capabilities of the automated chemical synthesis platform against the hardware index; and
(e) execute the instruction set on an automated chemical synthesis platform if it meets the required hardware capabilities.

The individual steps (a) to (e) correspond to the method steps described above.

The controller may be a general-purpose computer configured to perform the above steps. Examples of suitable general purpose computers include desktop computers, laptop or notebook computers, tablets and smart phones.

The controller may be local to (directly connected with) the automated chemical synthesis platform. Alternatively, the controller may by remote to (in a different location to) the automated chemical synthesis platform. In such cases, the controller may communicate with the automated chemical synthesis platform over a network (e.g. over the internet).

The controller may be implemented in a distributed computing environment. For example, the automated chemical synthesis platform may communicate with a cloud-based control system.

The controller may be a single entity. Alternatively, individual processing tasks may carried out by separate entities. For examples, a single processing unit may carry out the initial tagging step (a) and send the results to a separate proceeding unit to carry out one or more of the remaining control steps (b) to (e).

*Computer Program*

The invention also proves a data-processing device for an automated chemical synthesis platform comprising:

(a) means for receiving a synthetic procedure in natural language;
(b) means for tagging the synthetic procedure with *Subject, Action* and *Modifier* tags;
(c) means for producing a machine-readable instruction set from the tagged synthetic procedure; and
(d) means for generating a hardware index from the instruction set, and optionally
(e) means for checking the hardware capabilities of the automated chemical synthesis platform against the hardware index.

The invention also provides a computer program which, when the program is executed on a computer, cause the computer to carry out the steps of:

(a) tagging a synthetic procedure in natural language with *Subject, Action* and *Modifier* tags;
(b) producing a machine-readable instruction set from the tagged synthetic procedure; and
(c) generating a hardware index from the instruction set, and optionally
(d) checking the hardware capabilities of an automated chemical synthesis platform against the hardware index.

The invention also provides a computer-readable storage medium comprising instructions which, when executed by computer, cause the computer to carry out the steps of:

(a) tagging a synthetic procedure in natural language with *Subject, Action* and *Modifier* tags;
(b) producing a machine-readable instruction set from the tagged synthetic procedure; and
(c) generating a hardware index from the instruction set, and optionally
(d) checking the hardware capabilities of an automated chemical synthesis platform against the hardware index.

That is, the invention also provides a computer-readable storage medium having stored thereon any computer program disclosed herein.

*Other Embodiments*

Each and every compatible combination of the embodiments described above is explicitly disclosed herein, as if each and every combination was individually and explicitly recited.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above.

***Experimental***

The synthesis of four compounds based on procedures are described below to exemplify the method of controlling an automated chemistry platform.

*General Experimental Remarks*

All reagents were purchased from commercial suppliers in highest available purity and were used as received unless otherwise stated. Anhydrous THF, $Et_2O$, acetonitrile and DCM were acquired from in-house solvent purification system and stored over molecular sieves before use; anhydrous DME was purchased from Sigma Aldrich and used as received. All NMR measurements were recorded on Bruker Avance III HD 600 spectrometer operating at 600, 151 and 565 MHz for $^1H$, $^{13}C$ and $^{19}F$, respectively, or Bruker Avance III 400 spectrometer operating at 400 and 100 MHz for $^1H$ and $^{13}C$ respectively. Unless otherwise noted, the samples for NMR experiments were prepared in $CDCl_3$. Spectra were collected

at 298 K, and chemical shifts are reported in ppm relative to TMS or residual solvent (for $^1$H NMR: $CDCl_3$ = δ 7.26 ppm, $CD_3CN$ = δ 1.94 ppm, $CD_3OD$ = δ 3.31 ppm, DMSO-$d_6$ = δ 2.50 ppm; for $^{13}$C NMR: $CDCl_3$ = δ 77.16 ppm, $CD_3CN$ = δ 1.32, 118.26 ppm, $CD_3OD$ = δ 49.00 ppm, DMSO-$d_6$ = δ 39.52 ppm). Multiplicities are given as s: singlet, d: doublet, t: triplet, q: quartet, m: multiplet, br s: broad singlet with coupling constants reported in Hz. The spectra were processed with the Bruker Topspin 3.5 software package. High resolution mass spectra were obtained on a Bruker maXis LC/MS.

The synthesis of lidocaine and the oxidation of menthol using DMP used a modified version of the *Chemputer* conductivity sensor described in *Steiner.* This enhanced conductivity sensor has improved dynamic range and contains an extra reference resistor (100 kΩ) in addition to the existing 10 MΩ one, with a MOSFET transistor to periodically switch between them. The MOSFET's state is determined by a digital signal coming from the sensor microcontroller. Addition of the 100 kΩ resistor is motivated by the high conductivity of both organic and aqueous phases with certain combinations of solvent and solute, e.g. when an ionic component is soluble in the organic phase.

In the off state, an effective resistance close to the original 10 MΩ is seen while in the on state current can flow through the 100 kΩ resistor, resulting in a much lower effective reference resistance. Scheme 1 shows the electronic circuit diagram for the conductivity sensor circuit.

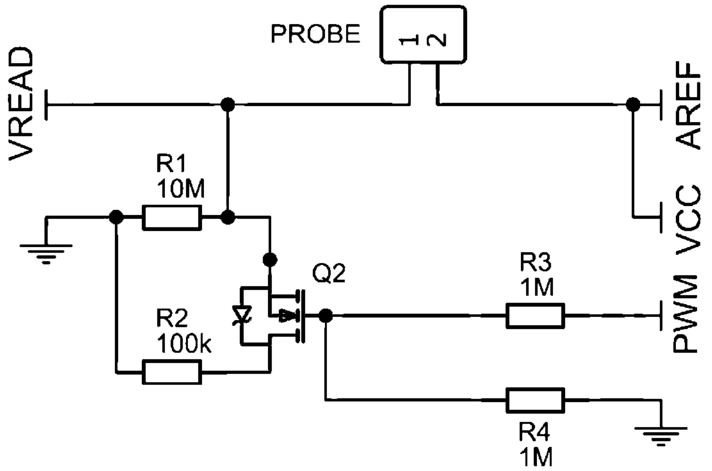


**Scheme 1:** Electronic schematic diagram of the enhanced conductivity sensor used for experiments used in this paper

Flash chromatography cartridges (EasyVarioFlash® series) of various sizes were used for drying and filtering organic solutions during the synthesises:

- 10 g empty cartridge (VWR, catalogue No. 554-3657)
- 150 g empty cartridge (VWR, catalogue No. 554-3925)

Drying reagents ($MgSO_4$, $Na_2SO_4$) were used as a mixture with sand; Celite® and silica gel were loaded manually and used as is.

For several filtering applications IDEX Bottom-of-the-Bottle™ filter (UHMWPE 10 μm, part No. A-446) were installed at the end of tubing in a reaction vessel using a standard 1/8" flangeless fittings.

*General Software Comments*

The example syntheses uses the *Chemputer* platform, with the instruction set in *XDL* and the hardware index in GraphML format. Additional technical details for the process are set out below.

**Conversion**

Further details on the process for converting the tagged synthetic procedure into an *XDL* file is set out below. Every operation has its own dedicated *XDL* converter function. The *XDL* converter function takes the sanitised operation and returns a list of *XDL* steps and a list of reagent names. For every operation the overall *XDL* step list and reagent list is extended with the *XDL* steps and reagent names generated from that operation.

Where the format for the instruction set is *XDL,* vessel assignment takes place. Vessel assignment uses the concept of a vessel chain. A vessel chain assumes there is one reaction mixture, moving through a linear series of vessels

throughout the procedure, with some non-linear exceptions. Each *XDL* step type has an associated vessel chain. Each link in the vessel chain has a list of rules, ordered by priority, to be used when assigning a vessel to that link in the vessel chain. Examples of the rules are shown in Table 4.

**Table 1:** Vessel assignment rules

| Rule | Description |
|---|---|
| prev | The vessel must be the same as the previous vessel in the vessel chain. |
| next | The vessel must be the same as the next vessel in the vessel chain. |
| heatchill | The vessel must be have heating/cooling capabilities (i.e. filter or reactor). |
| filter | The vessel must be a jacketed filter attached to a chiller. |
| reactor | The vessel must be a round bottom flask on a hotplate stirrer. |
| other | Type of vessel is not important, but it must be a different vessel to one preceding it in the chain. |
| group_to | Used for consecutive separation steps to reference the final vessel that the reaction mixture goes to after the final separation in the group. |
| rotavap | The vessel must be a rotary evaporator. |
| separator | The vessel must be a separator. |

As noted above, certain operations must be performed in specialised vessels. These specialised vessels are termed definite vessels. Examples of operations which must be performed in a definite vessel are filtration (which must be performed in a filter) or rotatory evaporation (which must be performed in a rotary evaporator).

The vessel assignment process involves the following steps:

1) Assign definite vessels (e.g. filter, separator).
2) Resolve undefined vessels based on rules (i.e. prev and next).
3) Add fake vessels at beginning and end in case there are no definite vessels there to allow assignment.
4) Try and resolve undefined vessels based on rules.
5) There is likely to be places in which rules create circular vessel dependence, for example a link with the rule 'next' followed by a link with the rule 'prev'. Resolve these by going back through the procedure and assigning the last defined vessel to every undefined link in the vessel chain.
6) Apply all the vessels in the vessel chain to the step list.
7) Add transfer steps in places where the vessel changes and the reaction mixture must be transferred.
8) Post-process the vessel list to resolve any exceptional situations in which the default vessel assignment is inappropriate.

During steps involving separations, i.e. washes and extractions, the automated platform must track which phase contains the product. Thus, the conversion step may comprise automated produce phase assignment. Where the instruction set is *XDL,* this information is computed and added to the *XDL* file during the synthesis conversion stage. A *XDL* method is used to track the contents of all vessels throughout the procedure and record the type and quantity of the solvents in the reaction mixture. The density of any common solvents in the reaction mixture can be compared with the density of the solvent added during the separation step to determine which phase will contain the product.

A phrase commonly encountered in synthetic procedures is "the reaction mixture was heated to reflux ...", without specifying a specific temperature. A similar approach to the automated product phase assignment process described above is also used to determine an appropriate heating temperature. When a heating step with the temperature specified as 'reflux' is encountered, the vessel contents are checked for common solvents. If any are found, the solvent's boiling point is used as the target temperature for heating. If a mixture of solvents is found, the boiling point of the most recently added solvent is used.

**Compilation**

An overview of the procedure for compiling the *XDL* code to the *Chemputer* is set out below.

1) Make a map of the closest vacuum, waste vessel, valve and inert gas vessels to every node in the graph.
2) Check that the graph contains all the components required by the procedure.

3) Check that flasks are present containing all the reagents specified.
4) Map abstract vessels in *XDL* to real vessels in the graph, and apply the component names from the graph to the Step objects.
5) Add in internal properties that are programmatically generated, for example waste vessels, reagent flask names and flask volumes.
6) Add steps implied by procedure, specifically steps to deal with the issue of keeping liquid in the top of the filter vessel until the point at which it is filtered, and cleaning the *Chemputer* backbone between different liquid transfers.
7) Add specific volumes to steps where required, taken from the properties of nodes in the graph, for example the volumes of the bottom and top of a filter module. Also use tabulated solvent boiling points to determine what temperature to heat to during CleanVessel steps.
8) Tidy up procedure. This involves removing any excessive backbone cleaning added during step 5, making sure no stirrers keep going after their vessels become empty.
9) Finally, any warnings picked up during the process are logged and the user is told that the *XDL* object is or is not ready for execution.

**Hardware mapping:** The first stage of the *Chemputer* compilation procedure is hardware mapping. This involves finding nodes in the graph corresponding to components declared in the *XDL* and converting the abstract component names in the *XDL* step objects to node names from the graph. As well as directly mapping declared vessels, some 'under the hood' vessels are mapped as well, for example waste vessels, inert gas vessels, etc. These are vessels which do not have to be declared in the *XDL* file as the execution module finds them in the graph and adds them automatically. The same hardware mapping procedure is used later in the procedure after other steps have been added.

**Implied steps:** Two aspects of automated synthesis are specific to the *Chemputer* hardware and so are not included in the *XDL* file. The first is the cleaning of the liquid backbone between steps involving liquid transfer to avoid cross-contamination between different steps of the procedure. The second is filling the bottom segment of the filter module so that the reaction mixture can be kept for long periods of time in the top part, described in the code as 'filter dead volume handling'. Stirring is also handled semi-automatically.

**Backbone cleaning:** Backbone cleaning is carried out by a step called CleanBackbone which moves a given solvent from its flask to all the waste containers attached to valves on the backbone. When to perform this step and what solvent to use are decided automatically by the *XDL* execution module according to the following rules.

CleanBackbone steps are added after any step involving liquid transfer through the backbone. The solvent to use is decided according to the reagents used in the procedure. If the step involves a common solvent, that solvent is used to clean the backbone, otherwise the last encountered common solvent is used. Additionally, if the next step will use a different solvent for cleaning according to these rules, another CleanBackbone step is added with this solvent so that the backbone is ready for this upcoming step.

A final check is carried out after this procedure which removes any pointless backbone cleans, for example between additions of the same reagent, or between a Filter and a Dry step.

**Filter dead volume handling:** The filter dead volume refers to the space below the filter in the filter module, which must be filled when not filtering to ensure that the reaction mixture stays in the top. *XDL* supports two methods of doing this.

In the inert gas method, a constant flow of inert gas is supplied to the filter bottom, creating enough pressure to keep liquid in the top. This is supported in *XDL* by automatically adding steps connecting the filter vessel to inert gas supply before any reagents are added, and disconnecting before a filtration or before liquid should be removed from the filter module. This method is especially useful in syntheses that require an inert atmosphere, since the flow of inert gas serves to remove any oxygen in the reactor headspace or dissolved in the reaction mixture.

The inert gas method can cause problems with foaming in some scenarios, and not all fume hoods have an inert gas line, so another method for handling dead volume is also supported. In this alternative method the bottom of the filter module is filled with a compatible solvent before any reagents are added to the top; this solvent is removed before performing a filtration or removing the reaction mixture from the filter module. This is achieved by two *XDL* steps: AddFilterDeadVolume and RemoveFilterDeadVolume. These use the same procedure as the backbone cleaning algorithm to determine the appropriate solvent at each step.

**Stirring:** Stirring is included in the procedure by setting the stir attribute of certain steps to True. A step with stirring specified will start stirring, and generally not stop stirring at the end of its execution. A step with stirring specified as False will stop stirring at the beginning of its execution. This works well to ensure that the right vessels are always being stirred. For example, an addition step can specify that stirring should begin, and then this stirring will continue even if a step that has nothing to do with stirring, like wait, is used. If a step explicitly states it should not be stirred stirring will be stopped before the step starts.

The only problem with this algorithm is that it means vessels can keep stirring long after they are no longer being used if there is no step in the procedure with stir set to False. To avoid this, the *XDL* execution module adds StopStir steps

whenever a vessel loses scope (becomes empty).

The *XDL* execution module also adds simple steps to set the stir speed of all stirrers to the default stirring speed at the start of the procedure. This speed can be altered at any step in the procedure using the stir_speed attribute of certain steps.

*Example 1: Lidocaine*

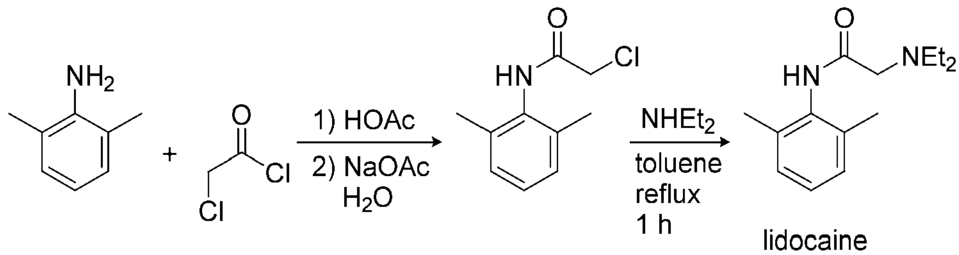


lidocaine

**Scheme 2:** Preparation of lidocaine

Lidocaine is used as a local anaesthetic and to treat arrhythmia and epilepsy (*Slaughter*).

The published synthesis for lidocaine is a simple two-step procedure involving the formation of an α-chloroamide intermediate and its subsequent nucleophilic substitution reaction with diethylamine (*Reilly*). These steps map in a straightforward fashion to the process diagram illustrated in Figure 3a.

**Literature procedure: N-(2,6-Dimethylphenyl)chloroacetamide**

2,6-Dimethylaniline (3.0 mL, 2.9 g, 24.4 mmol) is added to 15 mL of glacial acetic acid in a 125-mL Erlenmeyer flask followed by chloroacetyl chloride (2.0 mL, 2.85 g, 25.1mmol) and 25 mL of half-saturated aqueous sodium acetate. Precipitation of the amide is virtually instantaneous. The product is stirred thoroughly with 60 mL of cold water and isolated by vacuum filtration. It should be pressed as dry as possible in the Buchner funnel and used immediately in the next step.

**Literature procedure: 2-(Diethylamino)-N-(2,6-dimethylphenyl)acetamide (lidocaine)**

The amide is placed in a 50-mL round-bottom flask containing diethylamine (7.5 mL, 5.29 g, 72.5 mmol) and 25 mL of toluene and refluxed for one hour. The reaction mixture is cooled to room temperature and transferred to a separatory funnel, where it is washed 4× with 50 mL portions of water to remove diethylamine hydrochloride and excess diethylamine. The organic layer is extracted with one 20-mL portion of 3 M hydrochloric acid and washed once with 20 mL of water. The combined aqueous extracts are placed in a 125-mL Erlenmeyer flask, cooled to 10 °C in an ice bath, and neutralized by addition of 3 M sodium hydroxide in portions with stirring while maintaining the temperature below 20 °C. The product separates as a granular white solid and is isolated by vacuum filtration. It is washed with cold water, pressed dry, and air-dried as long as possible.

**Revised procedure: N-(2,6-Dimethylphenyl)chloroacetamide**

2,6-Dimethylaniline (3.0 mL, 2.9 g, 24.4 mmol) is added to 15 mL of glacial acetic acid in a 125-mL Erlenmeyer flask followed by chloroacetyl chloride (2.0 mL, 2.85 g, 25.1mmol) and 25 mL of half-saturated aqueous sodium acetate. Precipitation of the amide is virtually instantaneous. The product is stirred thoroughly with 60 mL of cold water and isolated by vacuum filtration. It should be pressed as dry as possible in the Buchner funnel and used immediately in the next step.

**Revised procedure: 2-(Diethylamino)-N-(2,6-dimethylphenyl)acetamide (lidocaine)**

The amide is placed in a 50-mL round-bottom flask containing diethylamine (7.5 mL, 5.29 g, 72.5 mmol) and 25 mL of toluene and refluxed for one hour. The reaction mixture is cooled to room temperature and transferred to a separatory funnel, where it is washed 4× with 50 mL portions of water to remove diethylamine hydrochloride and excess diethylamine. The organic layer is extracted with one 20-mL portion of 3 M hydrochloric acid and extracted[1] once with 20 mL of water. The combined aqueous extracts are placed in a 125 mL Erlenmeyer flask, cooled to 10 °C in an ice bath, and neutralized by addition of 3 M sodium hydroxide in portions with stirring while maintaining the temperature below 20 °C. The product

separates as a granular white solid and is isolated by vacuum filtration. It is washed with cold water, pressed dry, and air-dried as long as possible.

### Summary of changes

Additions in the revised procedure are indicated by underlining, and deletions are indicated by strikethrough.

[1] - *Washed* is changed to *extracted* to indicate that product phase is changed to aqueous and the organic phase can be discarded.

### Natural Language Output

The revised synthetic procedure was converted to the natural language output, shown below.

1) Add glacial acetic acid (15 mL) to filter.
2) Add 2,6-Dimethylaniline (3 mL) to filter.
3) Add chloroacetyl chloride (2 mL) to filter.
4) Add half-saturated aqueous sodium acetate (25 mL) to filter.
5) Heat/Chill filter to 10°C with stirring.
6) Add water (60 mL) to filter.
7) Stir filter for 60 mins at 250.
8) Stop heating/chilling filter.
9) Filter contents of filter.
10) Dry contents of filter for 60 mins.
11) Add diethylamine (7.5 mL) to filter.
12) Add toluene (25 mL) to filter.
13) Heat/Chill filter to 110.6°C for 60 mins.
14) Heat/Chill filter to 25°C with stirring.
15) Wash contents of filter with water (4 x 50 mL). Transfer waste phase (bottom) to None and product phase (top) to separator.
16) Extract contents of separator with 3 M hydrochloric acid (1 x 20 mL). Transfer waste phase (top) to separator and product phase (bottom) to filter.
17) Extract contents of separator with water (1 x 20 mL). Transfer waste phase (top) to None and product phase (bottom) to filter.
18) Heat/Chill filter to 10°C with stirring.
19) Add 3 M sodium hydroxide (20 mL) to filter.
20) Filter contents of filter.
21) Wash solid in filter with water (20 mL).
22) Dry contents of filter for 60 mins.
23) Dry contents of filter for 3 hrs.

### Execution of synthesis

The procedure was automatically scaled by 1.5. The generated XDL file is shown in Annex 1(A). The hardware graph is shown in Figure 6.

Based on the procedure described by the *XDL* file, the *Chemputer* operated the backbone pumps and valves to automatically transfer acetic acid solvent to the jacketed filter module - which the system had identified as a suitable reactor - followed by 2,6-dimethylaniline, chloroacetic acid, and saturated sodium acetate. During the process, the system correctly found points at which two chemicals are mixed and controlled stirring appropriately to ensure proper mixing. Based on the *XDL* instructions, the *Chemputer* then performed a filtration and routed the filtrate into a waste container. The next step was executed similarly by adding diethylamine and toluene solvent, heating the jacketed filter up to reflux using a circulation chiller to effect the substitution reaction, and using the liquid-liquid separation module to perform an acidic extraction with an aqueous hydrochloric acid solution. The detection of the liquid-liquid phase boundary is facilitated by a conductivity sensor exploiting the high conductivity of the aqueous phase compared to the organic phase. Finally, the lidocaine is precipitated from the aqueous solution in the jacketed filter by addition of sodium hydroxide solution, filtered, and dried in the jacketed filter under vacuum.

**Results**

The *Chemputer* successfully executed the synthesis based on the extracted instruction set and hardware requirements. When run at 150% scale, the yield of lidocaine is 3.75-4.57 g (43-53%) for three attempts.

**$^1$H NMR** (600 MHz, $CDCl_3$): δ 8.91 (br s, 1H), 7.11-7.06 (m, 3H), 3.22 (s, 2H), 2.68 (q, J = 7.1 Hz, 4H), 2.23 (s, 6H), 1.14 (t, J = 7.1 Hz, 6H). **$^{13}$C NMR** (151 MHz, $CDCl_3$): δ 170.3, 135.1, 134.0, 128.2, 127.1, 57.6, 49.0, 18.6, 12.7.

*Example 2: Dess-Martin Periodinane (DMP)*

Dess-Martin periodinane (DMP), is a versatile oxidation reagent that is prized for its specificity and functional group tolerance, despite its relatively high price and moisture sensitivity. Both the preparation and use of this reagent, as well as its precursor, 2-iodoxybenzoic acid (IBX), have been the subject of recent reproducibility debates (*Tojo & Fernández*).

*Organic Syntheses* contains a detailed preparation of DMP starting from 2-iodobenzoic acid (*Boeckman*), however, the present automated synthesis uses a newer reference for the preparation of the IBX intermediate that uses potassium monopersulfate (oxone) instead of potassium bromate as oxidant (*Frigerio*). The oxidising capacity of the product DMP was assessed by oxidising menthol according to the procedure of Reed. An outline of the overall synthesis is shown in Scheme 3.

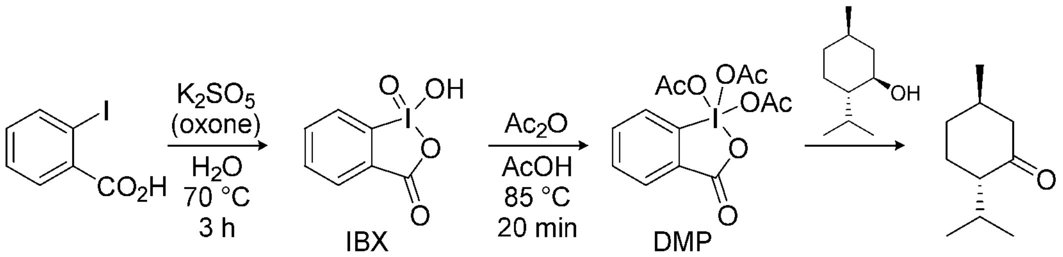


| Step yield (literature) | 79-81% | 75% | (95% efficient) |
|---|---|---|---|

**Scheme 3:** Preparation of DMP and oxidation of menthol using DMP.

***Caution:*** IBX and DMP exhibit explosive behaviour when heated beyond 154 °C and 130 °C, respectively. In addition, at least IBX is known to be impact sensitive (*Boeckman*). We are not aware of any operations in the following manual as well as automated procedures that would trigger either explosive tendency. Nevertheless, it is strongly recommended that the procedure be carried out behind a safety shield and inside a fumehood with the sash lowered. Further, it is prudent to carry out the reaction in an isolated area to ensure the safety of other workers.

**Literature procedure: 1-Hydroxy-1,2-benziodoxol-3(1H)-one 1-Oxide (IBX)**

2-Iodobenzoic acid (50.0 g, 0.20 mol) was added all at once to a solution of Oxone (181.0 g, 0.29 mol, 1.3 equiv) in deionized water (650 mL, 0.45 M) in a 2 L flask. The reaction mixture was warmed to 70-73 °C over 20 min and mechanically stirred at this temperature for 3 h. The aspect of the mixture varies consistently during the reaction. The initial thick slurry coating the walls of the flask eventually becomes a finely dispersed, easy to stir suspension of a small amount of solid that sedimented easily upon stopping the stirring. The suspension was then cooled to 5 °C and left at this temperature for 1.5 h with slow stirring. The mixture was filtered through a medium porosity sintered-glass funnel, and the solid was repeatedly rinsed with water (6 × 100 mL) and acetone (2 × 100 mL). The white, crystalline solid was left to dry at rt for 16 h and weighed 44.8-45.7 g (79-81%).

**Literature procedure: 1,1,1-Triacetoxy-1,1-dihydro-1,2-benziodoxol-3(1H)-one (Dess-Martin Periodinane)**

*B. 1,1,1-Triacetoxy-1,1-dihydro-1,2-benziodoxol-3(1H)-one* ***(2)*** . A 1-L, three-necked, round-bottomed flask, equipped for magnetic stirring and fitted with an immersion thermometer, is charged with 88.2 g of the moist solid iodinane oxide, 150 mL of glacial acetic acid, and 300 mL of acetic anhydride. The flask is flushed with dry argon, and maintained under a dry argon atmosphere. Magnetic stirring is commenced, and the mixture is heated to 85 °C (internal temperature) over 30 min by means of an oil bath and kept at this temperature until all the solids dissolve (~20 min) to afford a colorless to clear yellow solution. Heating and stirring are discontinued and the reaction mixture is allowed to cool slowly to room

temperature in the oil bath for 24 hr. A large quantity of colorless crystals separate during this time. The resulting crystalline solids are isolated by careful vacuum filtration in the reaction vessel under argon using a fritted adapter followed by washing the solids with three 80 mL portions of anhydrous ether and subsequent vacuum filtration in the reaction vessel as above. Residual solvent is removed under vacuum affording 101.0 g (74% yield over 2 steps) of periodinane **2** as a white, free-flowing crystalline solid that is largely or completely soluble (slightly cloudy to clear solution) in chloroform and methylene chloride and is sufficiently pure (~95%) to be suitable for use in oxidations.

**Literature procedure: Oxidation of menthol to menthone using DMP**

1. Obtain a sample of 1.0 - 1.1g of Dess-Martin periodinane (424.15 g/mole, 2.5 mmol).
2. Add 10 mL of methylene chloride to the periodinane in the flask as well as a small magnetic stir bar. Stir this solution.
3. Prepare a solution of either .35g of (-)-menthol or (+)-menthol (156.27 g/mole, 2.2 mmol) and 8 mL of methylene chloride in a 50-mL beaker. Swirl to dissolve the menthol in the solvent.
4. Add this second solution to the periodinane solution using a liquid funnel through the neck of the round bottom flask. Rinse the funnel with 1 mL of methylene chloride.
5. Stir this reaction for 30 minutes at room temperature. At the end of the reaction period, dilute the reaction mixture with 50 mL of diethyl ether. Add the resulting suspension to 10 mL of saturated aqueous sodium bicarbonate and 2.5g (.010 mole) of sodium thiosulfate in a 250-mL beaker. Vigorously stir this mixture for 15 minutes with a magnetic stirrer.
6. Transfer this solution to a 125-mL separatory funnel and allow the aqueous layer and the organic layer to separate.
7. Wash the organic layer successively with 10 mL of deionized water and 10 mL of brine.
8. Drain the organic layer into a 125-mL Erlenmeyer flask and dry it over a small amount of anhydrous magnesium sulfate. After swirling for a few minutes, gravity filter the product into a 125-mL Erlenmeyer flask.
9. Remove the solvent from the product using a rotary evaporator.

**Revised procedure: 1-Hydroxy-1,2-benziodoxol-3(1H)-one 1-Oxide (IBX)**

2-lodobenzoic acid (50.0 g, 0.20 mol) was added all at once to a solution of Oxone (181.0 g, 0.29 mol, 1.3 equiv) in deionized water (650 mL, 0.45 M) in a 2 L flask with vigorous stirring.[1] The reaction mixture was warmed to 70-73 °C over 20 min and mechanically stirred at this temperature for 3 h. The aspect of the mixture varies consistently during the reaction. The initial thick slurry coating the walls of the flask eventually becomes a finely dispersed, easy to stir suspension of a small amount of solid that sedimented easily upon stopping the stirring. The suspension was then cooled to 5 °C and left at this temperature for 1.5 h with slow stirring. The mixture was filtered through a medium porosity sintered-glass funnel, and the solid was repeatedly rinsed with water (6 × 100 mL) and acetone (2 × 100 mL). The white, crystalline solid was left to dry at rt for 2[2] h and weighed 44.8-45.7 g (79-81%).

**Revised procedure: 1,1,1-Triacetoxy-1,1-dihydro-1,2-benziodoxol-3(1H)-one (Dess-Martin Periodinane)**

A 1-L, three-necked, round-bottomed flask, equipped for magnetic stirring and fitted with an immersion thermometer, is charged with 56 g of the moist solid iodinane oxide, 96 mL of glacial acetic acid, and 192 mL of acetic anhydride. The flask is flushed with dry argon, and maintained under a dry argon atmosphere. Magnetic stirring is commenced, and the mixture is heated to 85°C (internal temperature) over 30 min by means of an oil bath, and kept at this temperature until all the solids dissolve (~20 min) to afford a colorless to clear yellow solution. Heating and stirring are discontinued and the reaction mixture is allowed to cool slowly to room temperature in the oil bath for 24 hr. A large quantity of colorless crystals separate during this time. The resulting crystalline solids are isolated by careful vacuum filtration in the reaction vessel under argon using a fritted adapter followed by washing the solids with three 80 mL portions of anhydrous ether and subsequent vacuum filtration in the reaction vessel as above.

**Revised procedure: Oxidation of menthol to menthone using DMP**

A solution of menthol (31.25 g in 500 mL DCM) was added to the DMP product and stirred for 30 min. The reaction was quenched by adding 234 mL of 25% aqueous sodium thiosulfate and 234 mL of saturated sodium bicarbonate solution and stirring vigorously for 30 min. The organic layer was extracted with ether (700 mL), washed twice with saturated sodium bicarbonate (2 × 468 mL) and dried over magnesium sulfate, then evaporated to give the crude product.

### Summary of changes

The three steps in this experiment have been sourced from independent literature procedures. As a result, we had to rescale the quantities of starting material in the second and third steps to match the expected yield of the first and second steps, respectively. Step 3 was adapted from an instruction list intended for an undergraduate laboratory experiment.

[1] - We specified *vigorous stirring* in step 1 because the iodobenzoic acid starting material is tends to get trapped in a thick foam otherwise.

[2] - Since step 2 required *moist* starting material, the drying time of product in step 1 was shortened from the original 16 hours to 2 hours.

### Execution of synthesis

The generated *XDL* files, representing the entire process, were joined together and the resulting XDL file is shown in Annex 1(B). The hardware graph is shown in Figure 7. The individual operations map to the process diagram illustrated in Figure 3b. The synthesis was executed on the *Chemputer* platform.

### Results

The menthol/menthone product mixture weighs 3.05 g (76% recovery). The mixture comprises menthone (53%) and menthol (47%), giving an oxidation efficiency of 53% as compared to 59% calculated as the oxidation efficiency of excess benzyl alcohol in the literature procedure (*Boeckman*). Characterisation data for the crude Dess-Martin reagent are listed below. They are in agreement with literature values (*Boeckman*).

In a separate experiment, the Dess-Martin reagent was isolated after the second step by dissolving in dichloromethane followed by filtering and evaporating the resulting solution. This procedure gave a yield of 5.52 g (13.0 mmol, 52%) of the DMP as a while solid.

**$^1$H NMR for DMP** (600 MHz, $CDCl_3$): $\delta$ 8.32-8.27 (m, 2H), 8.09-8.06 (m, 1H), 7.90 (t, J = 7.4 Hz, 1H), 2.32 (s, 3H), 1.99 (s, 6H). **$^{13}$C NMR for DMP** (151 MHz, $CDCl_3$): $\delta$ 175.8, 174.1, 166.2, 142.4, 135.8, 133.9, 131.9, 126.6, 126.1, 77.2, 20.5, 20.4.

*Example 3: 1,3-Bis(2,6-diisopropylphenyl)-2-fluoroimidazolium tetrafluoroborate (AlkylFluor)*

The four step sequence leading to chloroimidazolium salt **S3** is based on the literature procedure of *Tang.* The procedure, shown in Scheme 4, was performed in a single run. The procedure used a pre-made solution of 2,6-diisopropylaniline in MeOH with catalytic amount of HOAc, a premade TMSCl solution in EtOAc, a commercially supplied 1.6 M KO*t*Bu solution in THF (Acros Organics #428791000) and a premade 2M 1,1,1,2,2,2-hexachloroethane solution in THF. The original procedure was scaled down with respect to 10 ml (53 mmol) of 2,6-diisopropylaniline with modified solvent volume for washing procedures to ensure appropriate washing for the expected amount of the material.

The final step of the AlkylFluor synthesis (Scheme 4b) was based on the literature procedure of *Goldberg* and performed separately using chloroimidazolium salt **S3** obtained from the automatic run and commercially supplied KF and $KBF_4$ thoroughly dried before use. The procedure was scaled down with respect to 1 g (2.15 mmol) of 1,3-bis(2,6-diisopropylphenyl)-2-chloroimidazolium chloride (**S3**) with modified solvent volume for washing procedures to ensure appropriate washing for the expected amount of the material. *AlkylFluor's* precursors (*Mendoza-Espinosa*) and mechanism of action (*Neumann*) have been documented elsewhere.

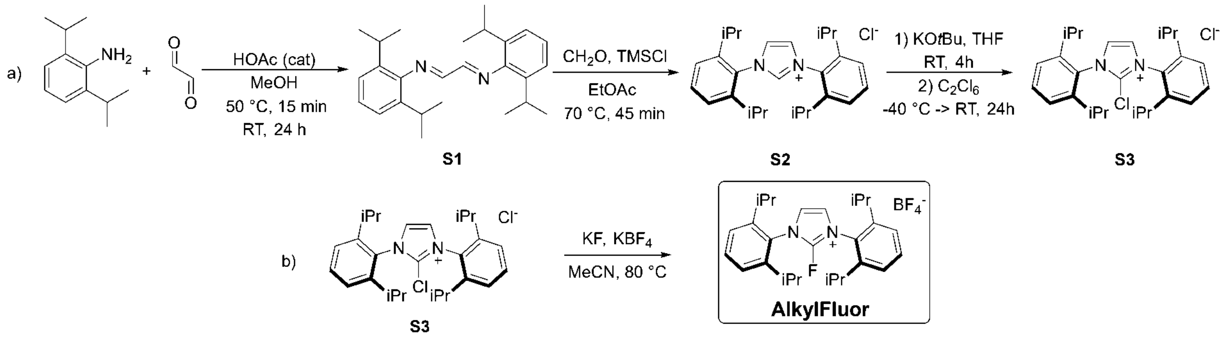


**Scheme 4:** a) Preparation of the 1,3-bis(2,6-diisopropylphenyl)-2-chloroimidazolium chloride (S3); b) Preparation of 1,3-Bis(2,6-diisopropylphenyl)-2-fluoroimidazolium tetrafluoroborate (AlkylFluor).

**Literature procedure: N,N'-1,4-Bis(2,6-diisopropylphenyl)-1,4-diaza-butadiene (S1)**

In air, to a solution of 2,6-diisopropylaniline (197 g, 1.00 mol, 2.00 equiv) and HOAc (1.0 mL, 0.018 mol, 0.035 equiv) in 250 mL of MeOH at 50 °C in a flask was added a solution of glyoxal (73 g, 40% in water, 0.50 mol, 1.0 equiv) in 250 mL of MeOH. The reaction mixture was stirred at 50 °C for 15 min and then stirred at 23 °C for 10 h. The reaction mixture was filtered. The filter cake was washed with MeOH (3 × 100 mL) and dried in vacuo to afford 169 g of compound **S1** as a yellow solid (90% yield).

**Literature procedure: N,N'-1,3-Bis(2,6-diisopropylphenyl)imidazolium chloride (S2)**

In air, to N,N'-1,4-bis(2,6-diisopropylphenyl)-1,4-diaza-butadiene **(S1)** (226 g, 0.600 mol, 1.00 equiv) and paraformaldehyde (18.1 g, 0.603 mol, 1.03 equiv) in 5.4 L of EtOAc in a flask at 70°C was added a solution of TMSCl (76.5 mL, 0.603 mol, 1.03 equiv) in 80 mL of EtOAc dropwise over 45 min with vigorous stirring. The reaction mixture was stirred at 70 °C for 2 h. After cooling to 10 °C with stirring, the reaction mixture was filtered. The filter cake was washed with EtOAc (3 × 500 mL) and dried in vacuo to afford 220 g of compound **S2** as a colourless solid (86% yield).

**Literature procedure: N,N'-1,3-Bis(2,6-diisopropylphenyl)-2-dichloroimidazolium chloride (S3)**

To N,N'-1,3-bis(2,6-diisopropylphenyl)imidazolium chloride **(S2)** (150 g, 353 mmol, 1.00 equiv) in 700 mL of THF in a flask at 23 °C was added KOtBu (47.4 g, 423 mmol, 1.20 equiv). The reaction mixture was stirred at 23 °C for 4 h. The reaction mixture was cooled to -40 °C and 1,1,1,2,2,2-hexachloroethane (100 g, 423 mmol, 1.20 equiv) was added. The reaction mixture was warmed to 23 °C and stirred at this temperature for 24 h. The reaction mixture was cooled to -40 °C and filtered. The filter cake was washed with cold THF (-20 °C, 3 x 100 mL) and toluene (6 × 100 mL). It was then dissolved in $CH_2Cl_2$ (500 mL) and filtered through a pad of Celite (10 g) eluting with $CH_2Cl_2$ (3 × 50 mL). The filtrate was concentrated under reduced pressure to afford 131 g of compound **S3** as a colorless solid (81% yield).

**Revised procedure: 1,3-Bis(2,6-diisopropylphenyl)-2-fluoroimidazolium tetrafluoroborate (AlkylFluor)**

All reagents were thoroughly dried before use. Under nitrogen atmosphere, 1,3-bis(2,6-diisopropylphenyl)-2-chloroimidazolium chloride **(S3)** (21.0 g, 45.7 mmol, 1.00 equiv.), potassium fluoride (7.97 g, 137 mmol, 3.00 equiv.), and potassium tetrafluoroborate (28.8 g, 229 mmol, 5.00 equiv.) were suspended in dry acetonitrile (300 mL) in a 350 mL pressure flask. The mixture was heated at 80 °C for 16 hours with vigorous stirring. The reaction mixture was cooled to room temperature, then filtered through a pad of Celite, eluting with dichloromethane (3 × 25 mL). The filtrate was concentrated in vacuo, and the residue was dissolved in dichloromethane (200 mL) and filtered again through Celite, eluting with dichloromethane (3 × 25 mL). The filtrate was concentrated in vacuo and the residual solid was washed with diethyl ether (3 × 15 mL) to afford the title compound as a colorless solid (18.8 g, 83%).

**Revised procedure: N,N'-1,4-Bis(2,6-diisopropylphenyl)-1,4-diaza-butadiene (S1)**

In air, to a solution of 2,6-diisopropylaniline (10 mL, 53.0 mmol, 2.00 equiv) and HOAc (0.2 mL, 3.5 mmol, 0.066 equiv) in 15 mL of MeOH at 50°C in a flask was added a solution of glyoxal (3.0 mL, 26.5 mmol, 1.0 equiv) in 15 mL of MeOH. The reaction mixture was stirred at 50°C for 15 min and then stirred at 23°C for 10 h. The reaction mixture was filtered. The

filter cake was washed with MeOH (3 × 15 mL) and dried in vacuo **at 75 mbar**[1] to afford 8.9 g of compound S1 as a yellow solid (90% yield).

**Revised procedure: N,N'-1,3-Bis(2,6-diisopropylphenyl)imidazolium chloride (S2)**

In air, to N,N'-1,4-bis(2,6-diisopropylphenyl)-1,4-diaza-butadiene **(S1)** (8.9 g, 23.9 mmol, 1.00 equiv) and paraformaldehyde (750 mg, 24.6 mmol, 1.03 equiv) was added 50 mL of EtOAc. A solution of TMSCl (3.3 mL, 24.6 mmol, 1.03 equiv) in 15 mL of EtOAc was then added at 70°C, dropwise, over 45 min, with vigorous stirring. The reaction mixture was stirred at 70°C for 2 h. After cooling to 10°C with stirring, the reaction mixture was filtered. The filter cake was washed with EtOAc (3 × 25 mL) and dried in vacuo at 75 mbar for 3 hours at 40°C[1] to afford 8.7 g of compound **S2** as a colourless solid (86% yield).

**Revised procedure: N,N'-1,3-Bis(2,6-diisopropylphenyl)-2-dichloroimidazolium chloride (S3)**

To N,N'-1,3-bis(2,6-diisopropylphenyl)imidazolium chloride(**S2**) (8.7 g, 21.7 mmol, 1.00 equiv) was added 5 mL of THF and a solution of KOtBu (2.92 g, 26.0 mmol, 1.20 equiv) in 16.3 mL of THF with vigorous stirring[2] at 23°C. The reaction mixture was vigorously[2] stirred at 23°C for 4 h. The reaction mixture was cooled to -35°C[3] and 2M 1,1,1,2,2,2-hexachloroethane solution (13 mL, 26.0 mmol, 1.20 equiv) was added. The reaction mixture was warmed to 23°C and stirred at this temperature for 24 h. The reaction mixture was cooled to -35°C[3] and filtered. The filter cake gummy precipitate[4] was washed with THF (3 × 10 mL) at -20°C and washed with toluene (6 x 10 mL) at 23°C[7]. It was then dissolved in $CH_2Cl_2$ (50 mL) and filtered through a pad of Celite (3 g) eluting with $CH_2Cl_2$ (3 × 25 mL). The filtrate was concentrated under reduced pressure to afford 5.0 g of compound **S3** as a colorless solid (81% yield).

**Revised procedure: 1,3-Bis(2,6-diisopropylphenyl)-2-fluoroimidazolium tetrafluoroborate (AlkylFluor)**

Under nitrogen atmosphere, potassium fluoride (380 mg, 6.45 mmol, 3.00 equiv.), potassium tetrafluoroborate (1.35 g, 10.75 mmol, 5.00 equiv.) and 1,3-bis(2,6-diisopropylphenyl)-2-chloroimidazolium chloride **(S3)** (1.0 g, 2.15 mmol, 1.00 equiv.) were added to a 25 mL pressure flask and dried for 3 hours at 80 °C[6]. The solids were suspended in 14.3 mL acetonitrile. The mixture was heated at 80 °C for 16 hours with vigorous stirring. The reaction mixture was cooled to room temperature, then filtered through a pad of Celite, eluting with dichloromethane (3 × 15 mL). The filtrate was concentrated in vacuo, and the residue was dissolved in dichloromethane (30 mL) and filtered again through Celite, eluting with dichloromethane (3 × 15 mL). It was then concentrated in vacuo, dried for 40 minutes at 75 mbar[1] and the residual solid was washed with diethyl ether (3 × 15 mL) to afford the title compound as a colorless solid (18.8 g, 83%).

**Summary of changes**

[1] - Extra drying to ensure no solvent contamination.
[2] - Vigorous stirring to a thick suspension is required.
[3] - Low temperature adjusted to the hardware used.
[4] - Keyword *gummy precipitate* was added to reduce the solvent withdrawing during the filtration to prevent filter clogging.
[5] - Extra temperature attribute to ensure washing done at room temperature.
[6] - Sufficient drying for 1,3-bis(2,6-diisopropylphenyl)-2-chloroimidazolium chloride **(S3).**
[7] - Explicit temperature given to ensure washing at ambient temperature.

**Execution of synthesis**

The procedure was automatically scaled by 1.5. The generated XDL file is shown in Annex 1(C) and 1(D). The hardware graph is shown in Figures 8 (steps 1-4) and 9 (step 5). The individual operations map to the process diagram illustrated in Figure 3c. The synthesis was executed on the *Chemputer* platform.

**Results**

Execution of the synthesis on the *Chemputer* platform obtaining *AlkylFluor* in 23% overall yield (75% average stepwise yield). This demonstrated that the text-to-molecule machinery is not limited to short syntheses, as the synthesis of *AlkylFluor* requires five distinct steps.

**$^1$H NMR for S3** (600 MHz, $CD_3CN$): δ. 8.32 (s, 2H), 7.72 (t, J = 7.8 Hz, 2H), 7.53 (d, J = 7.8 Hz, 4H), 2.38 - 2.29 (m, 4H), 1.27 (d, J = 6.8 Hz, 12H), 1.22 (d, J = 6.9 Hz, 12H). **$^{13}$C NMR for S3** (151 MHz, $CD_3CN$): δ. 146.3, 135.6, 133.8, 129.4, 127.7, 126.3, 30.2, 24.2, 23.5. **HRMS-ESI:** calc. for $[C_{27}H_{36}CN]^+$ 423.2562, found 423.2645.

**$^1$H NMR for AlkylFluor** (600 MHz, $CDCl_3$): δ 7.84 (d, J = 2.6 Hz, 2H), 7.65 (t, J = 7.8 Hz, 2H), 7.41 (d, J = 7.9 Hz, 4H), 2.49 (hept, J = 6.8 Hz, 4H), 1.34 (d, J = 6.7 Hz, 12H), 1.19 (d, J = 7.0 Hz, 12H). **$^{13}$C NMR for AlkylFluor** (151 MHz, $CDCl_3$): δ 145.0, 142.6 (d, $J_{CF}$ = 278.7 Hz),

132.7, 125.2, 124.9, 121.7 (d, $J_{CF}$ = 5.2 Hz), 29.1, 23.7, 23.2. **$^{19}$F NMR for AlkylFluor** (565 MHz, $CDCl_3$): δ -107.8, -152.9, -153.0. **HRMS-ESI:** calc. for $[C_{27}H_{36}FN]^+$ 407.2857, found 407.2979.

*Example 4: Polyoxometalate synthesis*

The literature synthesis of the polyoxometalate $(C_2H_8N)_8Na_3[W_{19}Mn_2O_{61}Cl(SeO_3)_2(H_2O)_2]Cl_2$ •$6H_2O$ was described by Symes *et al.*

## Literature procedure

An aqueous solution of $MnCl_2{\cdot}4H_2O$ (5.0 mL; 9.36 g in 450 mL of water) was added to the reaction vessel, followed by 4.0 mL of solution of $Na_2WO_4{\cdot}2H_2O$ (75 g), DMA·HCl (30 g) and $Na_2SeO_3$ (6 g) in 450 mL of water. To the mixture was added 1.50 mL HCl (2.32 M aqueous solution; 57.1 mL of concentrated HCl diluted with water to 300 mL). The mixture was stirred for 10 seconds and the stirring was stopped to allow crystals formation within 2 hours.

## Revised procedure

An Mn solution (5.0 mL; 9.36 g in 450 mL of water) was added to the reaction vessel, followed by of W solution 4.0ml of water. To the mixture was added 1.50 mL HCl (2.32 M aqueous solution; 57.1 mL of concentrated HCl diluted with water to 300 mL). The mixture was stirred for 10 seconds and the stirring was stopped to allow crystals formation within 2 hours.

## Summary of changes

Name of the solutions were changed to meet the limitations in the length of the device name when communicating over serial connection.

## Execution of synthesis

The synthesis was performed on both the *Clusterbot* platform and the *Chemputer,* from the same *XDL* file, shown in Annex 1(E). The hardware graph for the *Chemputer* platform is shown in 10 and the hardware graph for the *Clusterbot* platform is shown in Figure 11. Crystals were obtained from both syntheses and the structure was confirmed by X-ray diffraction.

## Results

Diffraction experiments were performed on a Rigaku Synergy. The unit cell obtained from the crystal $(C_2H_8N)_8Na_3[W_{19}Mn_2O_{61}Cl(SeO_3)_2(H_2O)_2]Cl_2{\cdot}6H_2O$ synthesised by *Clusterbot* was Monoclinic, space group C*2*/*m*, with unit cell parameters *a* = 35.09, *b* = 20.77, *c* = 18.07 Å, $\beta$ = 108.71°, *V* = 12475 Å$^3$. Unit cell parameters obtained from the *Chemputer* were the following: *a* = 35.17, *b* = 20.78, *c* = 18.04 Å, $\beta$ = 108.99°, *V* = 12467 Å$^3$. These parameters are close enough to the parameters previously published to confirm that the correct structure was obtained: Monoclinic, space group C2/*m*, *a* = 36.022(3), *b* = 20.6218(13), *c* = 17.8132(13) Å. $\beta$ = 110.322(5)°, V = 12408.7(16) Å3 (Symes *et al*).

These results demonstrate that the text-to-molecules machinery can be used to control chemical synthesis using different automated chemistry platforms.

## *References*

A number of publications are cited above in order to more fully describe and disclose the invention and the state of the art to which the invention pertains. Full citations for these references are provided below. The entirety of each of these references is incorporated herein.

BOECKMAN, R. K. at al., 2000, "The Dess-Martin Periodinane: 1,1,1-Triacetoxy-1,1-Dihydro-1,2-Benziodoxol-3(1h)-One", Organic Synthesis, Vol. 77, pp. 141.

FRIGERIO, M. et al., 1999, "A User-Friendly Entry to 2-lodoxybenzoic Acid (IBX)", The Journal of Organic Chemistry, Vol. 64, pp. 4537-4538.

GOLDBERG, N. W. et al., 2016, "AlkylFluor: Deoxyfluorination of Alcohols", Organic Letters, Vol. 18, pp. 6102-6104.

HAWIZY, L. et al, 2011, "ChemicalTagger: A tool for semantic text-mining in chemistry", Journal of Cheminformatics, Vol. 3, pp. 1-13.

MENDOZA-ESPINOSA, D. et al., 2010, "Synthesis of 4- and 4,5-Functionalized Imidazol-2-ylidenes from a Single 4,5-Unsubstituted Imidazol-2-ylidene", Journal of the America Chemical Society, Vol. 132, pp. 7264-7265.

NEUMANN, C. N., et al., 2016, "Concerted nucleophilic aromatic substitution with 19F- and 18F-", Nature, Vol. 534, pp. 369-373.

REED, N. A. et al., 2005, "Circular Dichroism Investigation of Dess-Martin Periodinane Oxidation in the Organic Chemistry Laboratory", Journal of Chemical Education, Vol. 82 .pp. 1053-1054.

REILLY, T. J., 1999, "The Preparation of Lidocaine", Journal of Chemical Education, Vol. 76, pp. 1557.

SLAUGHTER, L. A. et al, 2013, "Pharmacological Treatment of Neonatal Seizures:A Systematic Review", Journal of Child Neurology, Vol. 28, pp. 351-364.

STEINER, S. et al., 2019, "Organic synthesis in a modular robotic system driven by a chemical programming language", Science, Vol. 363, pp 1-8.

SYMES, M. D. et al., 2012, "Integrated 3D-printed reactionware for chemical synthesis and analysis." Nature Chemistry, Vol. 4, 349-354.

TANG, P. et al., 2011, "Deoxyfluorination of Phenols", Journal of the America Chemical Society, Vol. 133, pp. 11482-11484.

TOJO, G. & FERNÁNDEZ, M., 2010, "Oxidation of alcohols to aldehydes and ketones : a guide to current common practice" Springer.

***Annex 1 (XDL Code)***

*A) Generated XDL for Lidocaine Synthesis*

```
<Synthesis>
 <Hardware>
  <Component
   id="filter"
   type="filter" />
  <Component
   id="separator"
   type="separator" />
 </Hardware>

 <Reagents>
  <Reagent
   id="glacial acetic acid" />
  <Reagent
   id="2,6-Dimethylaniline" />
  <Reagent
   id="chloroacetyl chloride" />
  <Reagent
   id="half-saturated aqueous
sodium acetate" />
  <Reagent
   id="diethylamine" />
  <Reagent
   id="toluene" />
  <Reagent
   id="water" />
  <Reagent
   id="3 M hydrochloric acid" />
  <Reagent
   id="3 M sodium hydroxide" />
 </Reagents>

 <Procedure>
  <Add
   reagent="glacial acetic acid"
   vessel="filter"
   volume="15 mL"
   stir="False" />
  <Add
   reagent="2,6-Dimethylaniline"
   vessel="filter"
   volume="3 mL"
   stir="True" />
  <Add
   reagent="chloroacetyl chloride"
   vessel="filter"
   volume="2 mL"
   stir="True" />
  <Add
   reagent="half-saturated aqueous
sodium acetate"
   vessel="filter"
   volume="25 mL"
   stir="True" />
  <HeatChillToTemp
   vessel="filter"
   temp="10°C" />
  <Add
   reagent="water"
   vessel="filter"
   volume="60 mL"
   stir="True" />
  <Stir
   vessel="filter"
   time="60 mins" />
  <StopHeatChill
   vessel="filter" />
  <Filter
   filter_vessel="filter" />
  <Dry
   vessel="filter" />
  <Add
   reagent="diethylamine"
   vessel="filter"
   volume="7.5 mL"
   stir="False" />
  <Add
   reagent="toluene"
   vessel="filter"
   volume="25 mL"
   stir="True" />
  <HeatChill
   vessel="filter"
   temp="110.6°C"
   time="60 mins" />
  <HeatChillToTemp
   vessel="filter"
   temp="25°C" />
  <Transfer
   from_vessel="filter"
   to_vessel="separator"
   volume="all" />
  <Separate
   purpose="wash"
   from_vessel="separator"
   separation_vessel="separator"
   to_vessel="separator"
   solvent="water"
   product_bottom="False"
   solvent_volume="50 mL"
   n_separations="4" />
  <Separate
   purpose="extract"
   from_vessel="separator"
   separation_vessel="separator"
   to_vessel="filter"
```

```
    solvent="3 M hydrochloric acid"
    product_bottom="True"
    solvent_volume="20 mL"
    n_separations="1"
waste_phase_to_vessel="separator"
/>
  <Separate
    purpose="extract"
    from_vessel="separator"
    separation_vessel="separator"
    to_vessel="filter"
    solvent="water"
    product_bottom="True"
    solvent_volume="20 mL"
    n_separations="1" />
  <HeatChillToTemp
    vessel="filter"
    temp="10°C" />
  <Add
    reagent="3 M sodium hydroxide"
    vessel="filter"
    volume="20 mL"
    dispense_speed="10"
    stir="True" />
  <Filter
    filter_vessel="filter" />
  <WashSolid
    vessel="filter"
    solvent="water"
    temp="10°C"
    repeat="1" />
  <Dry
    vessel="filter" />
  <Dry
    vessel="filter"
    time="3 hrs" />
 </Procedure>

</Synthesis>
```

*B) Generated XDL for Dess-Martin Periodinane (DMP)Synthesis*

```
<Synthesis>
 <Hardware>
  <Component
   id="cartridge_MgSO4"
   type="cartridge"
   chemical="MgSO4" />
  <Component
   id="reactor"
   type="reactor" />
  <Component
   id="rotavap"
   type="rotavap" />
  <Component
   id="separator"
   type="separator" />
 </Hardware>

 <Reagents>
  <Reagent
   id="1 M HCl" />
  <Reagent
   id="1 M NaOH" />
  <Reagent
   id="benzylamine" />
  <Reagent
   id="dichloromethane" />
  <Reagent
   id="p-toluenesulfonyl chloride
dichloromethane solution" />
  <Reagent
   id="saturated aqueous NaCl
solution" />
  <Reagent
   id="triethylamine" />
 </Reagents>

 <Procedure>
  <Add
   reagent="benzylamine"
   vessel="reactor"
   volume="11.5 mL"
   stir="False" />
  <Add
   reagent="triethylamine"
   vessel="reactor"
   volume="15.5 mL"
   stir="True" />
  <Add
   reagent="dichloromethane"
   vessel="reactor"
   volume="250 mL"
   stir="True" />
  <HeatChillToTemp
   vessel="reactor"
   temp="3°C" />
  <Add
   reagent="p-toluenesulfonyl
chloride dichloromethane solution"
   vessel="reactor"
   volume="100 mL"
   time="45 mins"
   stir="True" />
  <Stir
   vessel="reactor"
   time="30 mins" />
  <HeatChillToTemp
```

```
 vessel="reactor"
 temp="18°C"
 active="False"
 continue_heatchill="False" />
<HeatChill
 vessel="reactor"
 temp="21°C"
 time="13 hrs" />
<Add
 reagent="1 M HCl"
 vessel="reactor"
 volume="250 mL"
 stir="True" />
<Transfer
 from_vessel="reactor"
 to_vessel="separator"
 volume="all" />
<Separate
 purpose="extract"
 from_vessel="separator"
 separation_vessel="separator"
 to_vessel="separator"
 product_bottom="True"
 solvent=""
 n_separations="1" />
<Separate
 purpose="wash"
 from_vessel="separator"
 separation_vessel="separator"
 to_vessel="separator"
 product_bottom="True"
 solvent="1 M HCl"
 solvent_volume="250 mL"
 n_separations="2" />
<Separate
 purpose="wash"
 from_vessel="separator"
 separation_vessel="separator"
 to_vessel="separator"
 product_bottom="True"
 solvent="1 M NaOH"
 solvent_volume="250 mL"
 n_separations="2" />
<Separate
 purpose="wash"
 from_vessel="separator"
 separation_vessel="separator"
 to_vessel="rotavap"
 product_bottom="True"
 solvent="saturated aqueous NaCl
solution"
 through="MgSO4"
 solvent_volume="250 mL"
 n_separations="1" />
<Evaporate
 rotavap_name="rotavap"
 temp="25°C"
 pressure="33.3305 mbar"
 time="30 mins"
 mode="auto" />
<Dry
 vessel="rotavap"
 time="6 hrs"
 vacuum_pressure="0.2666 mbar"
/>
</Procedure>

</Synthesis>
```

*C) Generated XDL for Synthesis of **S3***

```
<Synthesis
filter_dead_volume_method="solvent
">
 <Hardware>
  <Component
   id="cartridge_celite"
   type="cartridge"
   chemical="celite" />
  <Component
   id="filter"
   type="filter" />
  <Component
   id="rotavap"
   type="rotavap" />
 </Hardware>

 <Reagents>
  <Reagent
   id="2,6-diisopropylaniline and
HOAc MeOH solution" />
  <Reagent
   id="2M 1,1,1,2,2,2
hexachloroethane solution" />
  <Reagent
   id="CH2Cl2" />
  <Reagent
   id="EtOAc" />
  <Reagent
   id="KOtBu THF solution" />
  <Reagent
   id="MeOH" />
  <Reagent
   id="N,N'-1,3-bis(2,6-
diisopropylphenyl)imidazolium
chloride(S2)" />
  <Reagent
```

```
    id="N,N'-1,4-bis(2,6-
  diisopropylphenyl)-1,4-diaza-
  butadiene(S1)" />
    <Reagent
     id="THF" />
    <Reagent
     id="TMSCl EtOAc solution" />
    <Reagent
     id="glyoxal MeOH solution" />
    <Reagent
     id="paraformaldehyde" />
    <Reagent
     id="toluene" />
   </Reagents>

   <Procedure>
    <Add
     reagent="2,6-diisopropylaniline
  and HOAc MeOH solution"
     vessel="filter"
     volume="25.2 mL"
     stir="False" />
    <HeatChillToTemp
     vessel="filter"
     temp="50°C" />
    <Add
     reagent="glyoxal MeOH solution"
     vessel="filter"
     volume="18 mL"
     stir="True" />
    <Stir
     vessel="filter"
     time="15 mins" />
    <HeatChill
     vessel="filter"
     temp="23°C"
     time="10 hrs" />
    <Filter
     filter_vessel="filter" />
    <WashSolid
     vessel="filter"
     solvent="MeOH"
     volume="15 mL"
     repeat="3" />
    <Dry
     vessel="filter"
     vacuum_pressure="75 mbar" />
    <Add
     reagent="paraformaldehyde"
     vessel="filter"
     mass="0.75 g"
     stir="False" />
    <Add
     reagent="N,N'-1,4-bis(2,6-
  diisopropylphenyl)-1,4-diaza-
  butadiene(S1)"
     vessel="filter"
     mass="8.9 g"
     stir="False" />
    <Add
     reagent="EtOAc"
     vessel="filter"
     volume="50 mL"
     stir="False" />
    <HeatChillToTemp
     vessel="filter"
     temp="70°C" />
    <Add
     reagent="TMSCl EtOAc solution"
     vessel="filter"
     volume="18.3 mL"
     time="45 mins"
     stir="True"
     stir_speed="600 RPM" />
    <Stir
     vessel="filter"
     time="2 hrs" />
    <HeatChillToTemp
     vessel="filter"
     temp="10°C" />
    <Filter
     filter_vessel="filter" />
    <WashSolid
     vessel="filter"
     solvent="EtOAc"
     volume="25 mL"
     repeat="3" />
    <Dry
     vessel="filter"
     time="3 hrs"
     vacuum_pressure="75 mbar"
     temp="40°C" />
    <HeatChillToTemp
     vessel="filter"
     temp="23°C" />
    <Add
     reagent="N,N'-1,3-bis(2,6-
  diisopropylphenyl)imidazolium
  chloride(S2)"
     vessel="filter"
     mass="8.7 g"
     stir="False" />
    <Add
     reagent="THF"
     vessel="filter"
     volume="5 mL"
     stir="True"
     stir_speed="600 RPM" />
    <Add
     reagent="KOtBu THF solution"
     vessel="filter"
     volume="16.3 mL"
```

```
   stir="True"
   stir_speed="600 RPM" />
  <Stir
   vessel="filter"
   time="4 hrs"
   stir_speed="600 RPM" />
  <HeatChillToTemp
   vessel="filter"
   temp="-35°C" />
  <Add
   reagent="2M 1,1,1,2,2,2
hexachloroethane solution"
   vessel="filter"
   volume="13 mL"
   stir="True" />
  <HeatChillToTemp
   vessel="filter"
   temp="23°C" />
  <Stir
   vessel="filter"
   time="24 hrs" />
  <HeatChillToTemp
   vessel="filter"
   temp="-35°C" />
  <Filter
   filter_vessel="filter"
   anticlogging="True" />
  <WashSolid
   vessel="filter"
   solvent="THF"
   volume="10 mL"
   temp="-20°C"
   repeat="3"
   anticlogging="True" />
  <WashSolid
   vessel="filter"
   solvent="toluene"
   volume="10 mL"
   temp="23°C"
   repeat="6"
   anticlogging="True" />
  <Dry
   vessel="filter" />
  <Dissolve
   vessel="filter"
   solvent="CH2Cl2"
   volume="50 mL" />
  <FilterThrough
   from_vessel="filter"
   to_vessel="rotavap"
   through="celite"
   eluting_solvent="CH2Cl2"
   eluting_volume="25 mL"
   eluting_repeats="3" />
  <Evaporate
   rotavap_name="rotavap"
   temp="50°C"
   pressure="699 mbar"
   time="30 mins"
   mode="auto" />
 </Procedure>

</Synthesis>
```

*D) Generated XDL for Conversion of* ***S3*** *to AlkylFluor*

```
<Synthesis>
 <Hardware>
  <Component
   id="cartridge_celite"
   type="cartridge"
   chemical="celite" />
  <Component
   id="reactor"
   type="reactor" />
  <Component
   id="rotavap"
   type="rotavap" />
 </Hardware>

 <Reagents>
  <Reagent
   id="1,3-bis(2,6-
diisopropylphenyl)-2-
chloroimidazolium chloride" />
  <Reagent
   id="acetonitrile" />
  <Reagent
   id="dichloromethane" />
  <Reagent
   id="diethyl ether" />
  <Reagent
   id="potassium fluoride" />
  <Reagent
   id="potassium
tetrafluoroborate" />
 </Reagents>

 <Procedure>
  <Add
   reagent="potassium fluoride"
   vessel="reactor"
   mass="0.38 g"
   stir="False" />
  <Add
   reagent="potassium
tetrafluoroborate"
```

```
     vessel="reactor"
     mass="1.35 g"
     stir="False" />
    <Add
     reagent="1,3-bis(2,6-
diisopropylphenyl)-2-
chloroimidazolium chloride"
     vessel="reactor"
     mass="1 g"
     stir="False" />
    <Dry
     vessel="reactor"
     time="3 hrs"
     temp="80°C" />
    <Add
     reagent="acetonitrile"
     vessel="reactor"
     volume="14.3 mL"
     stir="False" />
    <HeatChill
     vessel="reactor"
     temp="80°C"
     time="16 hrs"
     stir_speed="600 RPM" />
    <HeatChillToTemp
     vessel="reactor"
     temp="25°C" />
    <FilterThrough
     from_vessel="reactor"
     to_vessel="rotavap"
     through="celite"

eluting_solvent="dichloromethane"
     eluting_volume="15 mL"
     eluting_repeats="3" />
    <Evaporate
     rotavap_name="rotavap"
     temp="50°C"
     pressure="426 mbar"
     time="30 mins"
     mode="auto" />
    <Dissolve
     vessel="rotavap"
     solvent="dichloromethane"
     volume="30 mL" />
    <FilterThrough
     from_vessel="rotavap"
     to_vessel="rotavap"
     through="celite"

eluting_solvent="dichloromethane"
     eluting_volume="15 mL"
     eluting_repeats="3" />
    <Evaporate
     rotavap_name="rotavap"
     temp="50°C"
     pressure="699 mbar"
     time="30 mins"
     mode="auto" />
    <Dry
     vessel="rotavap"
     time="40 mins"
     vacuum_pressure="75 mbar" />
    <WashSolid
     vessel="rotavap"
     solvent="diethyl ether"
     volume="15 mL"
     repeat="3" />
  </Procedure>

</Synthesis>
```

*E) Generated XDL for Synthesis of Polyoxyometalate*

```
<Synthesis>
 <Hardware>
  <Component
   id="reactor"
   type="reactor" />
 </Hardware>

 <Reagents>
  <Reagent
   id="HCl" />
  <Reagent
   id="Mn_Solution" />
  <Reagent
   id="W_Solution" />
 </Reagents>

 <Procedure>
  <StartStir vessel="reactor"
stir_speed="55" />
  <Add
   reagent="W_Solution"
   vessel="reactor"
   volume="5.0 mL"
  />
  <Add
   reagent="Mn_Solution"
   vessel="reactor"
   volume="4.0 mL"
  />
  <Add
   reagent="HCl"
   vessel="reactor"
   volume="1.50 mL"
  />
  <Stir vessel="reactor"
stir_speed="20" time="10" />
  <StopStir vessel="reactor" />
 </Procedure>
</Synthesis>
```

***Statements of Invention:***

The following numbered paragraphs contain statements of broad combinations of technical features in accordance with various aspect of the invention disclosed herein:

1. A method for controlling an automated chemical synthesis platform, the method comprising:

   (a) providing a synthetic procedure in natural language;
   (b) tagging the synthetic procedure with *Subject, Action* and *Modifier* tags;
   (c) producing a machine-readable instruction set from the tagged synthetic procedure;
   (d) generating a hardware index from the machine-readable instruction set; and
   (e) checking the hardware capability of the automated chemical synthesis platform against the hardware index; and optionally
   (f) executing the machine-readable instruction set on an automated chemical synthesis platform meeting the hardware capability.

2. The method of paragraph 1, wherein step (b) comprises using pattern matching to apply the *Action* and *Modifier* tags.

3. The method of paragraph 1 or 2, wherein step (b) comprises:

   (i) generating a list of candidate reagent names from the synthetic procedure;
   (ii) checking the list of candidate reagent names against a list of commonplace reagent names;
   (iii) applying a *Reagent* tag to candidate reagent names which match a commonplace reagent name; and
   (iv) applying the *Subject* tag to text comprising the *Reagent* tag using pattern matching.

4. The method of any of paragraphs 1 to 3, wherein step (b) comprises:

   (i) generating a list of candidate reagent names from the synthetic procedures;
   (ii) extracting word fragments of 2 to 4 characters from the candidate reagent names;
   (iii) calculating the probability that the word fragments are part of a chemical compound name or part of normal text;

(iv) applying a *Reagent* tag to candidate reagent names which meet a probability threshold; and
(v) applying the *Subject* tag to text comprising the *Reagent* tag using pattern matching.

5. The method of paragraph 4, wherein the probability is calculated using a Naïve Bayes classifier.

6. The method of any of paragraphs 1 to 5, wherein step (c) comprises:

(a) combining a tagged *Action* with a tagged *Modifier* to give a pair based on their relative location in a sentence; and
(b) combining an *Action* and *Modifier* pair with a tagged *Subject* to give an operation using pattern matching.

7. The method of any of paragraphs 1 to 6, wherein the machine-readable instruction set is in a mark-up language, such as *XDL.*

8. The method of any of paragraphs 1 to 7, comprising in step (c), outputting the machine-readable instruction set in natural language, and optionally cross-checking the natural language output with the natural language synthetic procedure, and optionally correcting a discrepancy between the natural language synthetic procedure and the natural language input.

9. The method of any of paragraphs 1 to 8, wherein the hardware index is in graph format.

10. The method of paragraph 9, wherein step (d) comprises:

(a) providing a template graph describing the automated chemical synthesis platform hardware; and
(b) populating the template graph using the instruction set.

11. The method of any of paragraphs 1 to 10, comprising adapting the machine-readable instruction set to meet the hardware capabilities of the automated chemical synthesis platform, such as automatically scaling the procedure to meet the capacity of the automated chemical synthesis platform.

12. The method of any of paragraphs 1 to 11, wherein the automated synthesis platform is a general automated synthesis platform, such as a *Chemputer.*

13. A controller for an automated chemical synthesis platform, wherein the controller is configured to:

(a) tag a synthetic procedure in natural language with *Subject, Action* and *Modifier* tags;
(b) produce a machine-readable instruction set from the tagged synthetic procedure;
(c) generate a hardware index from the instruction set;
(d) check the hardware capability of the automated chemical synthesis platform against the hardware index; and
(e) execute the instruction set on an automated chemical synthesis platform if it meets the required hardware capability.

## Claims

**1.** A computer-implemented method for controlling an automated chemical synthesis platform, the method comprising:

(a) providing a synthetic procedure in natural language;
(b) tagging the synthetic procedure with *Subject, Action* and *Modifier* tags;
(c) producing a machine-readable instruction set from the tagged synthetic procedure;
(d) generating a hardware index from the machine-readable instruction set; and
(e) checking the hardware capability of the automated chemical synthesis platform against the hardware index.

**2.** The method of claim 1, wherein step (b) comprises using pattern matching to apply the *Action* and *Modifier* tags.

**3.** The method of claim 1 or 2, wherein step (b) comprises:

(i) generating a list of candidate reagent names from the synthetic procedure;

(ii) checking the list of candidate reagent names against a list of commonplace reagent names;
(iii) applying a *Reagent* tag to candidate reagent names which match a commonplace reagent name; and
(iv) applying the *Subject* tag to text comprising the *Reagent* tag using pattern matching.

**4.** The method of any of claims 1 to 3, wherein step (b) comprises:

(i) generating a list of candidate reagent names from the synthetic procedures;
(ii) extracting word fragments of 2 to 4 characters from the candidate reagent names;
(iii) calculating the probability that the word fragments are part of a chemical compound name or part of normal text, optionally wherein the probability is calculated using a Naive Bayes classifier;
(iv) applying a *Reagent* tag to candidate reagent names which meet a probability threshold; and
(v) applying the *Subject* tag to text comprising the *Reagent* tag using pattern matching.

**5.** The method of any of claims 1 to 4, wherein step (c) comprises:

(a) combining a tagged *Action* with a tagged *Modifier* to give a pair based on their relative location in a sentence; and
(b) combining an *Action* and *Modifier* pair with a tagged *Subject* to give an operation using pattern matching.

**6.** The method of any of claims 1 to 5, wherein the machine-readable instruction set is in a mark-up language, such as *XDL*.

**7.** The method of any of claims 1 to 6, comprising in step (c), outputting the machine-readable instruction set in natural language, and optionally cross-checking the natural language output with the natural language synthetic procedure, and optionally correcting a discrepancy between the natural language synthetic procedure and the natural language input.

**8.** The method of any of claims 1 to 7, wherein the hardware index is in graph format, optionally wherein step (d) comprises:

(a) providing a template graph describing the automated chemical synthesis platform hardware; and
(b) populating the template graph using the instruction set.

**9.** The method of any of claims 1 to 8, comprising adapting the machine-readable instruction set to meet the hardware capabilities of the automated chemical synthesis platform, such as automatically scaling the procedure to meet the capacity of the automated chemical synthesis platform.

**10.** The method of any of claims 1 to 9, further comprising:
(f) providing the machine-readable instruction set for execution on an automated chemical synthesis platform meeting the hardware capability.

**11.** A computer-readable storage medium comprising instructions for controlling an automated chemical synthesis platform, wherein the instructions, when executed by a computer, cause the computer to carry out the steps of:

(a) tagging a synthetic procedure in natural language with *Subject, Action* and *Modifier* tags;
(b) producing a machine-readable instruction set from the tagged synthetic procedure; and
(c) generating a hardware index from the instruction set, and
(d) checking the hardware capabilities of an automated chemical synthesis platform against the hardware index.

**12.** The computer-readable storage medium of claim 11, wherein the instructions, when executed by a computer, cause the computer to carry out the step of:
(e) providing the machine-readable instruction set for execution on an automated chemical synthesis platform meeting the hardware capability.

**13.** A controller for an automated chemical synthesis platform, wherein the controller is configured to:

(a) tag a synthetic procedure in natural language with *Subject, Action* and *Modifier* tags;
(b) produce a machine-readable instruction set from the tagged synthetic procedure;

(c) generate a hardware index from the instruction set; and
(d) check the hardware capability of the automated chemical synthesis platform against the hardware index.

**14.** The controller of claim 13, wherein the controller is further configured to:
(e) provide the machine-readable instruction set for execution on an automated chemical synthesis platform meeting the hardware capability.

**15.** The method of any one of claims 1 to 10, the computer-readable storage medium of claim 11 or 12, or the controller of claim 13 or 14, wherein the machine-readable instruction set is for execution on an automated chemical synthesis platform, optionally wherein the automated synthesis platform is a general automated synthesis platform, such as a *Chemputer*.

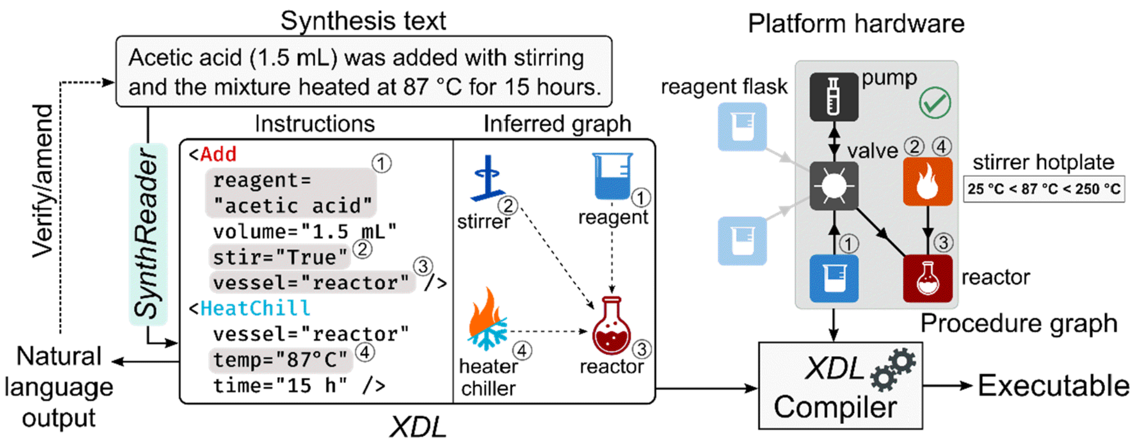


*Figure 1*

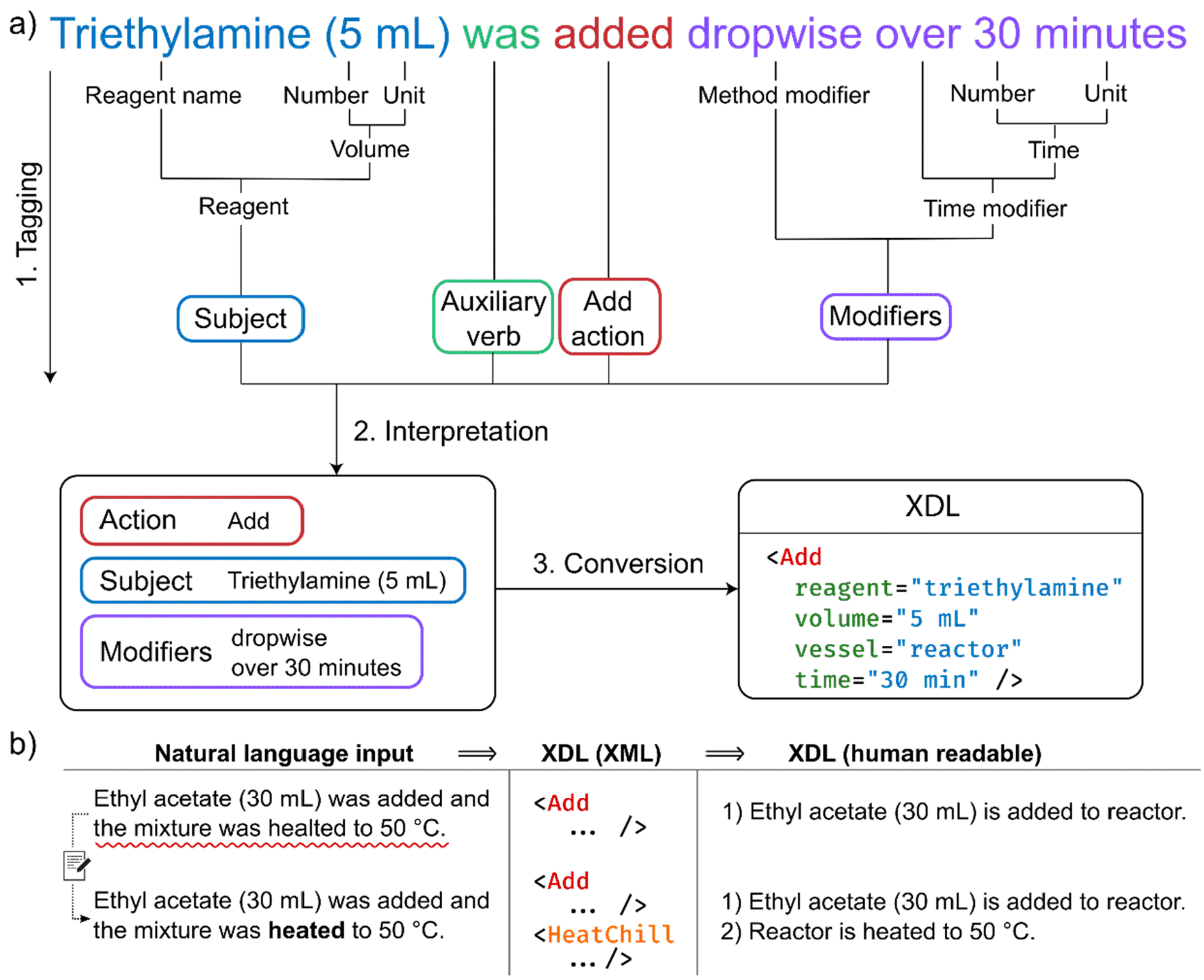


*Figure 2*

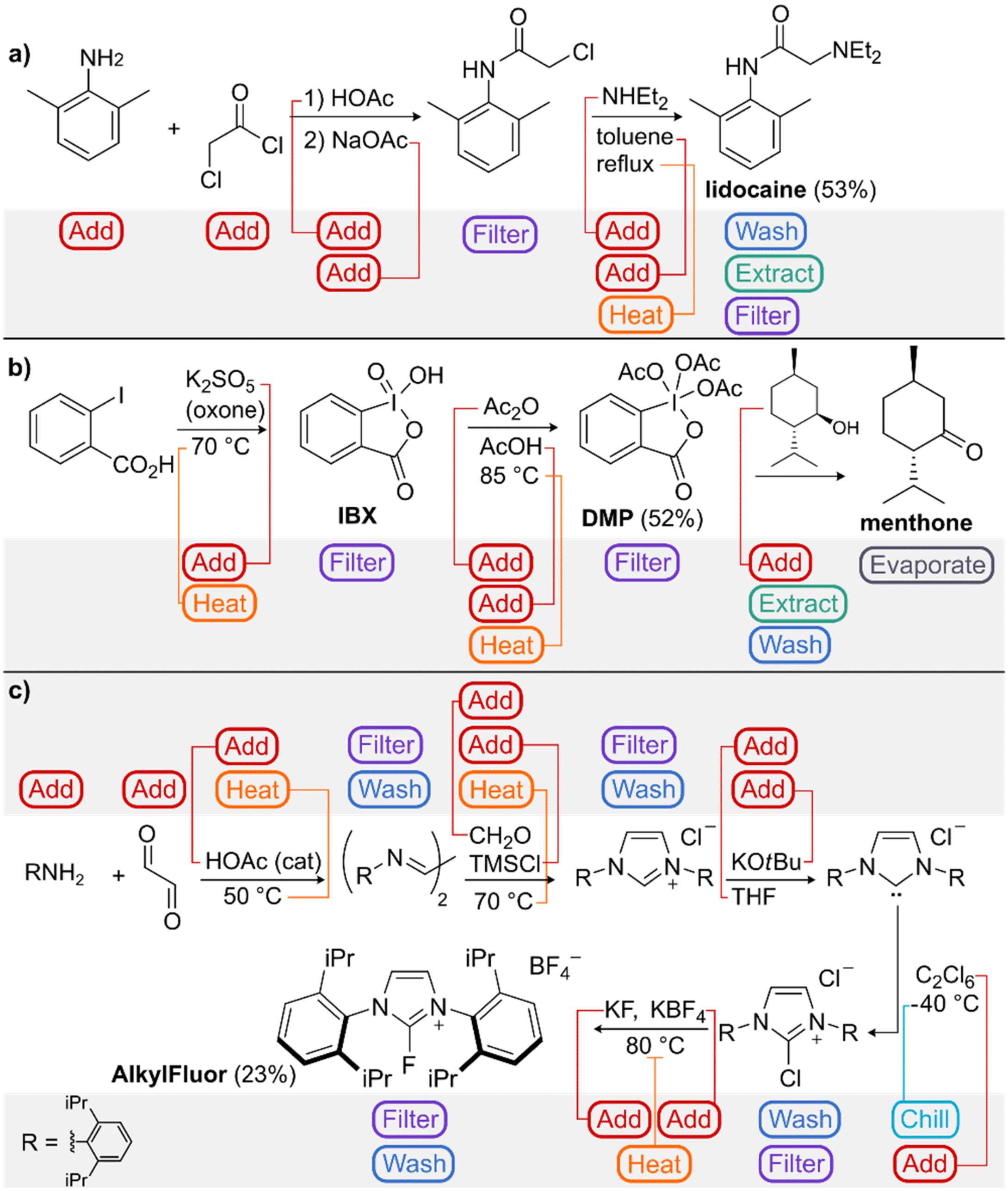
a)
1) HOAc
2) NaOAc
$NHEt_2$
toluene
reflux
lidocaine (53%)
Add
Add
Add
Add
Filter
Add
Add
Heat
Wash
Extract
Filter
b)
$K_2SO_5$
(oxone)
70 °C
IBX
$Ac_2O$
AcOH
85 °C
DMP (52%)
menthone
Add
Heat
Filter
Add
Add
Heat
Filter
Add
Extract
Wash
Evaporate
c)
Add
Add
Add
Heat
Filter
Wash
Add
Add
Heat
Filter
Wash
Add
Add
HOAc (cat)
50 °C
$CH_2O$
TMSCl
70 °C
KOtBu
THF
$C_2Cl_6$
-40 °C
KF, $KBF_4$
80 °C
AlkylFluor (23%)
Filter
Wash
Add
Add
Heat
Wash
Filter
Chill
Add

*Figure 3*

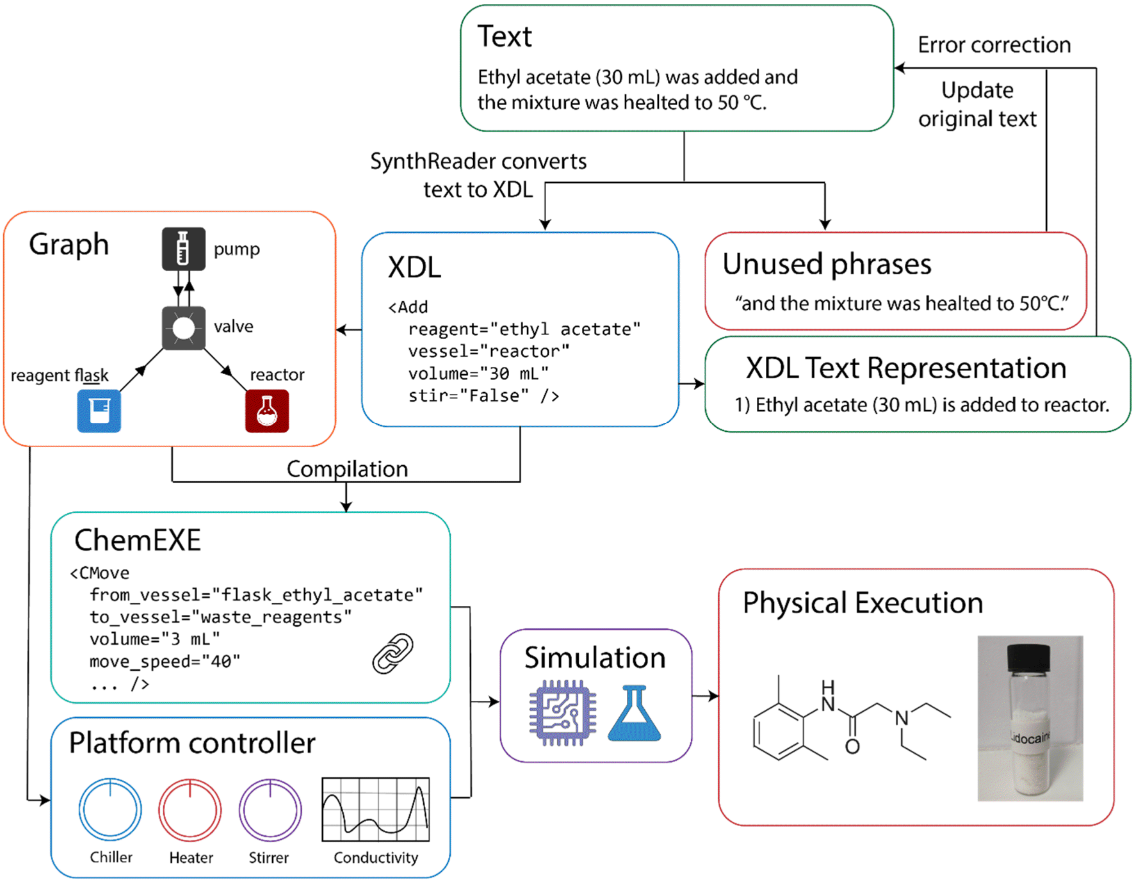


***Figure 4***

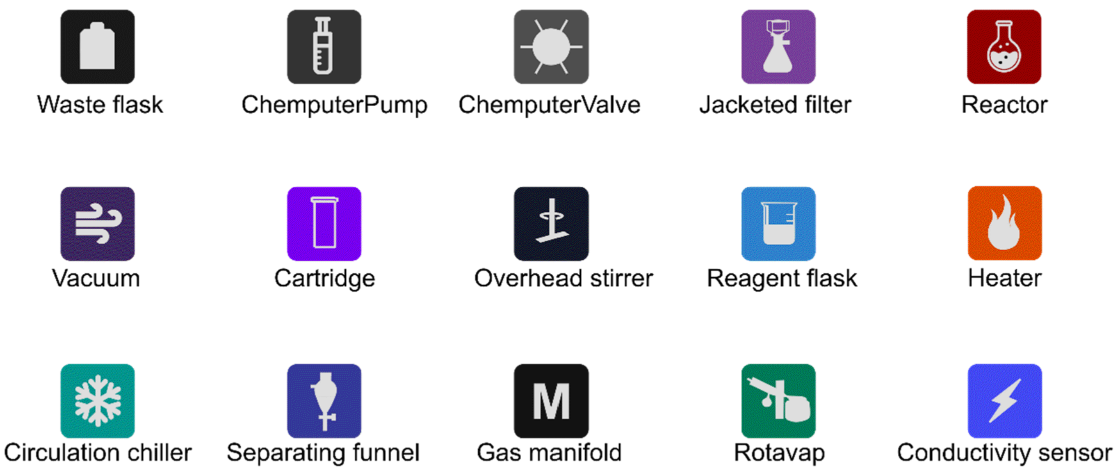


***Figure 5***

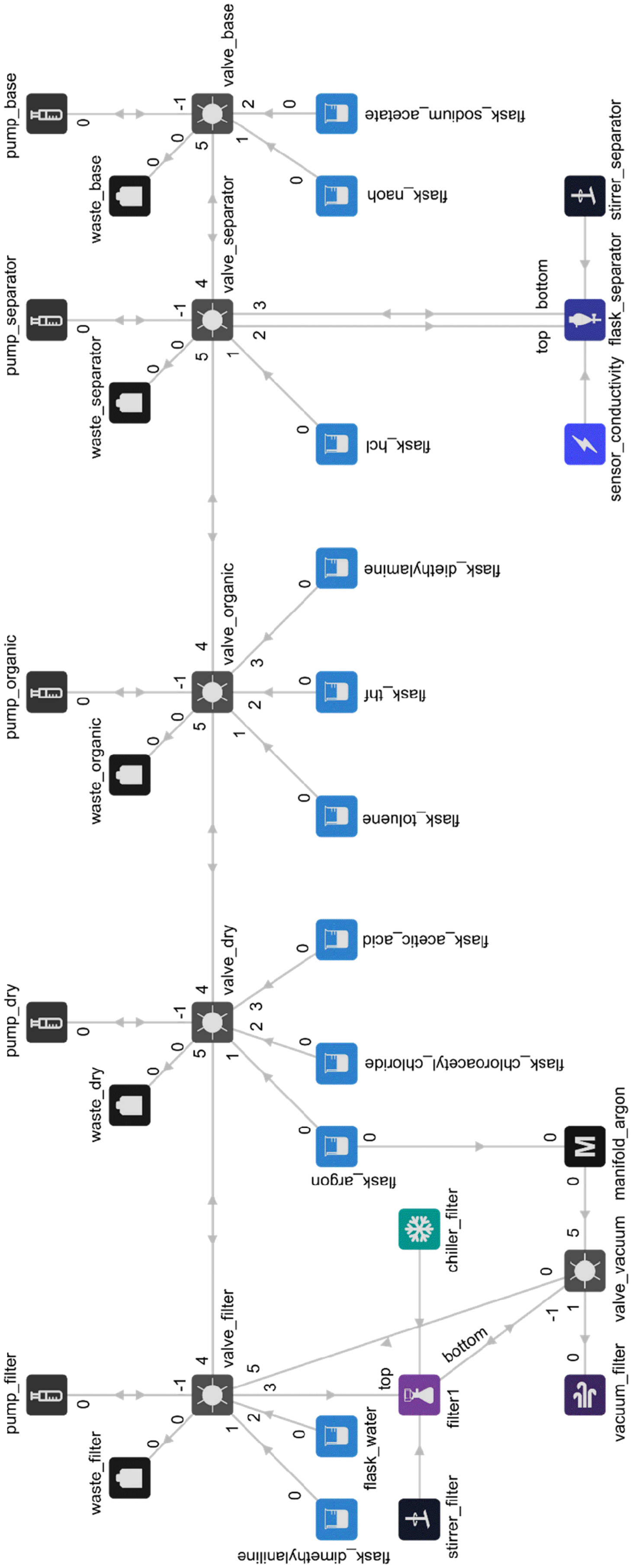
pump_base
waste_base
valve_base
flask_sodium_acetate
flask_naoh
pump_separator
waste_separator
valve_separator
flask_hcl
flask_separator
sensor_conductivity
stirrer_separator
top
bottom
pump_organic
waste_organic
valve_organic
flask_diethylamine
flask_thf
flask_toluene
pump_dry
waste_dry
valve_dry
flask_acetic_acid
flask_chloroacetyl_chloride
flask_argon
manifold_argon
chiller_filter
valve_vacuum
pump_filter
waste_filter
valve_filter
flask_water
flask_dimethylaniline
filter1
stirrer_filter
vacuum_filter
top
bottom

*Figure 6*

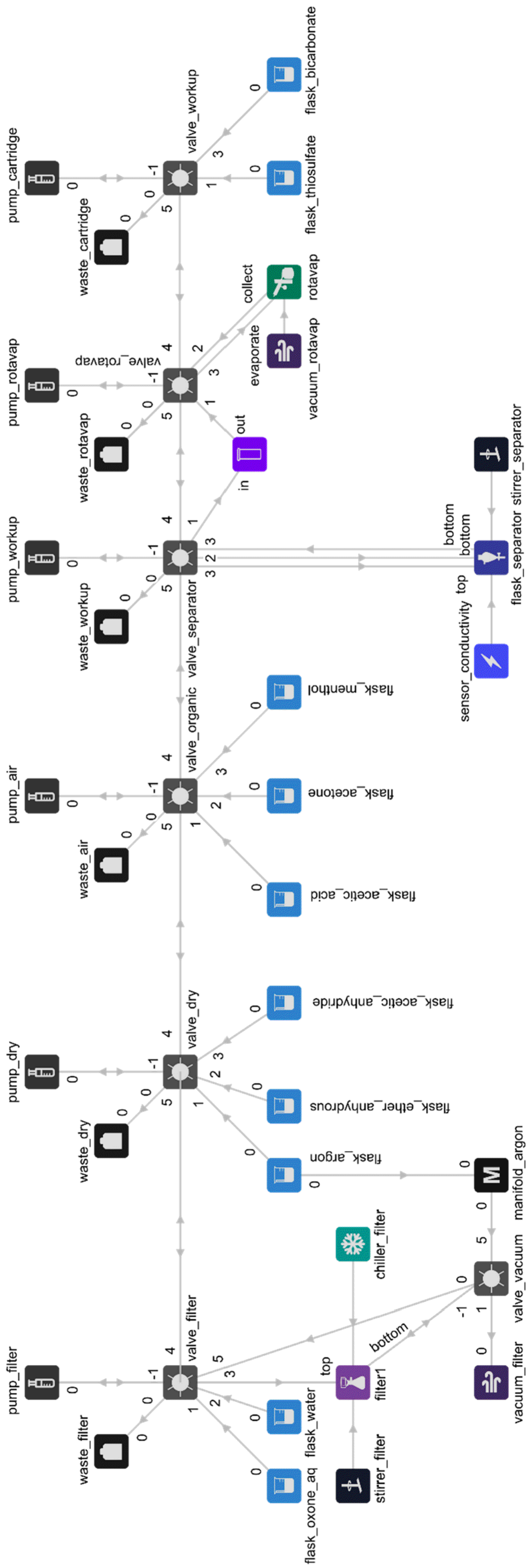
pump_filter
waste_filter
valve_filter
flask_oxone_aq
flask_water
stirrer_filter
top
filter1
bottom
chiller_filter
vacuum_filter
valve_vacuum
manifold_argon
pump_dry
waste_dry
valve_dry
flask_argon
flask_ether_anhydrous
flask_acetic_anhydride
pump_air
waste_air
valve_organic
flask_acetic_acid
flask_acetone
flask_menthol
pump_workup
waste_workup
valve_separator
sensor_conductivity
top
bottom
bottom
flask_separator
stirrer_separator
in
out
pump_rotavap
waste_rotavap
valve_rotavap
evaporate
collect
vacuum_rotavap
rotavap
pump_cartridge
waste_cartridge
valve_workup
flask_thiosulfate
flask_bicarbonate

*Figure 7*

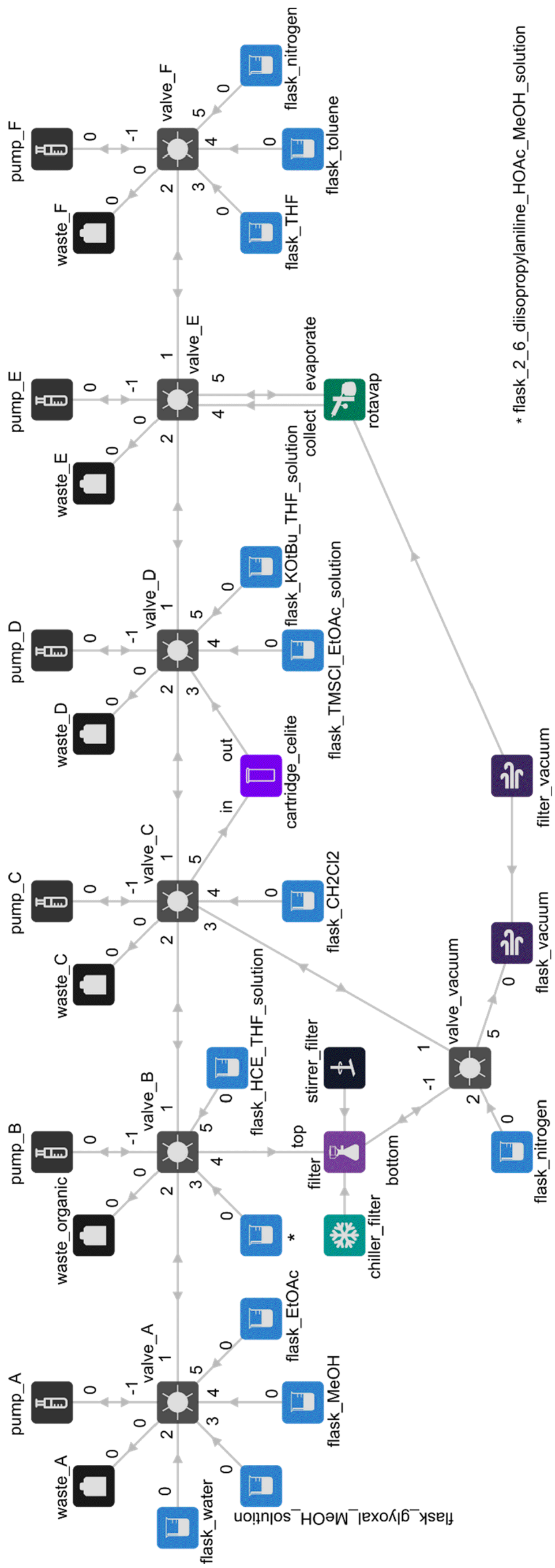
pump_A
waste_A
valve_A
flask_water
flask_glyoxal_MeOH_solution
flask_MeOH
flask_EtOAc
pump_B
waste_organic
valve_B
flask_HCE_THF_solution
*
stirrer_filter
filter
top
bottom
chiller_filter
pump_C
waste_C
valve_C
flask_CH2Cl2
cartridge_celite
in
out
valve_vacuum
flask_nitrogen
flask_vacuum
filter_vacuum
pump_D
waste_D
valve_D
flask_KOtBu_THF_solution
flask_TMSCl_EtOAc_solution
pump_E
waste_E
valve_E
rotavap
collect
evaporate
pump_F
waste_F
valve_F
flask_THF
flask_toluene
flask_nitrogen
* flask_2_6_diisopropylaniline_HOAc_MeOH_solution

*Figure 8*

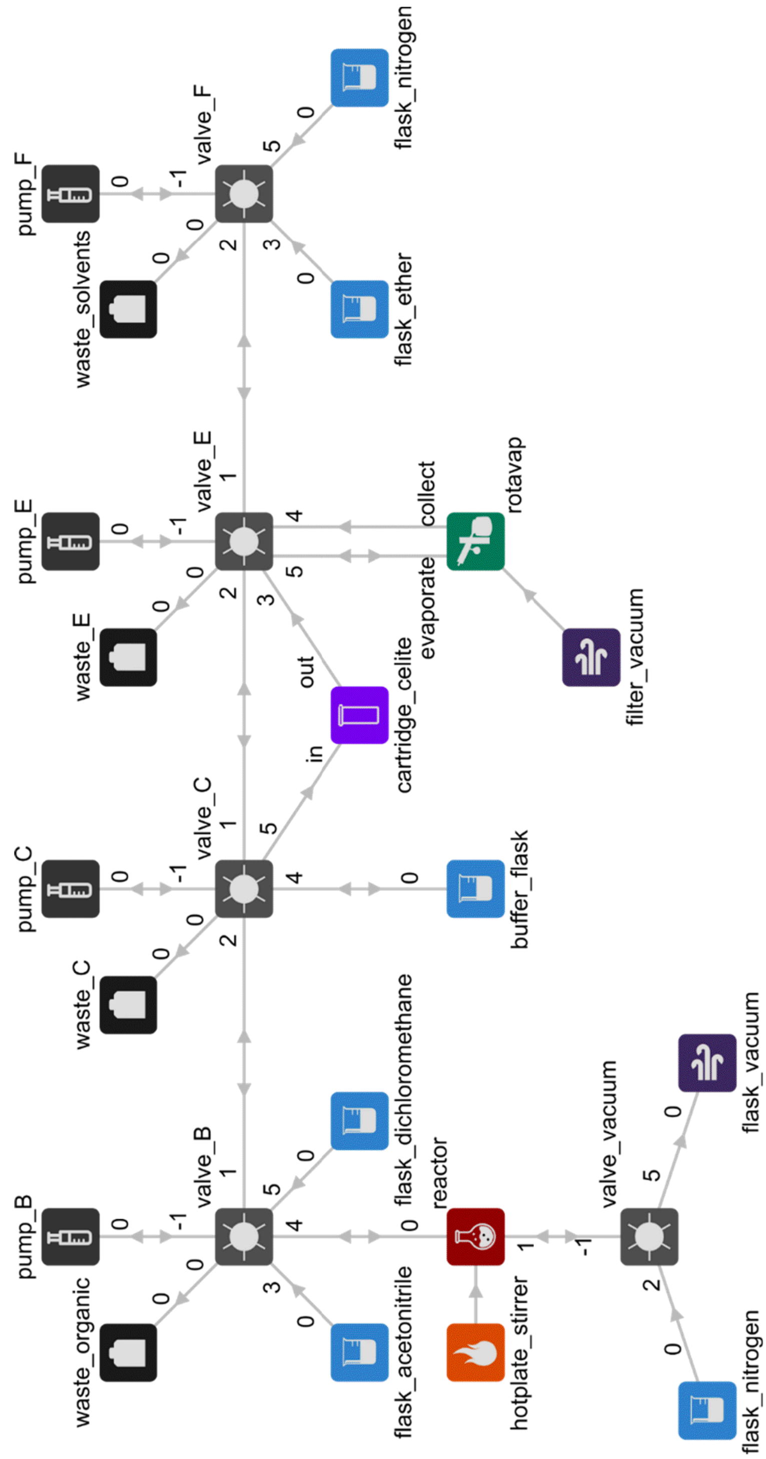
pump_B
waste_organic
valve_B
flask_acetonitrile
flask_dichloromethane
reactor
hotplate_stirrer
valve_vacuum
flask_nitrogen
flask_vacuum
pump_C
waste_C
valve_C
buffer_flask
cartridge_celite
in
out
pump_E
waste_E
valve_E
rotavap
collect
evaporate
filter_vacuum
pump_F
waste_solvents
valve_F
flask_ether
flask_nitrogen
-1
0
1
2
3
4
5

*Figure 9*

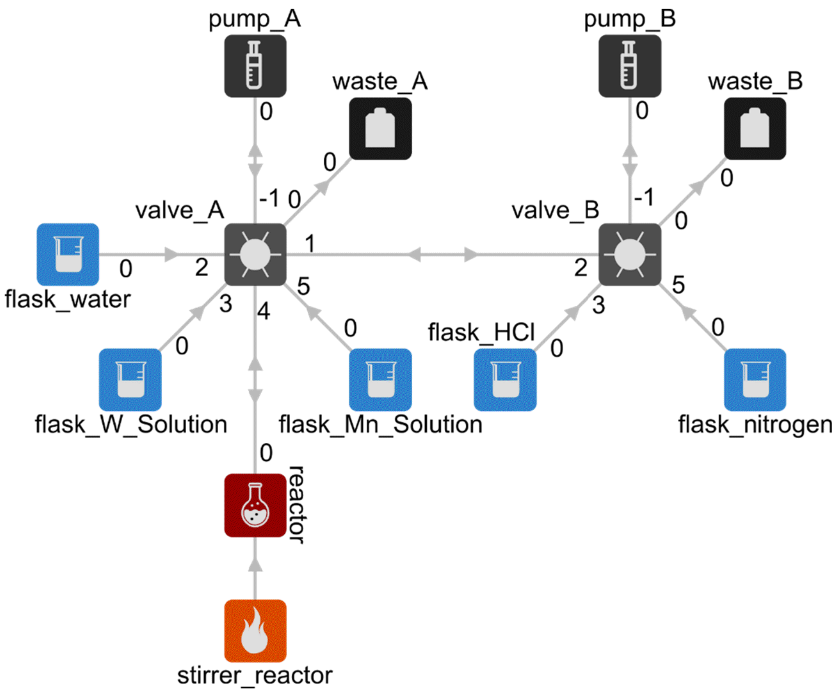


***Figure 10***

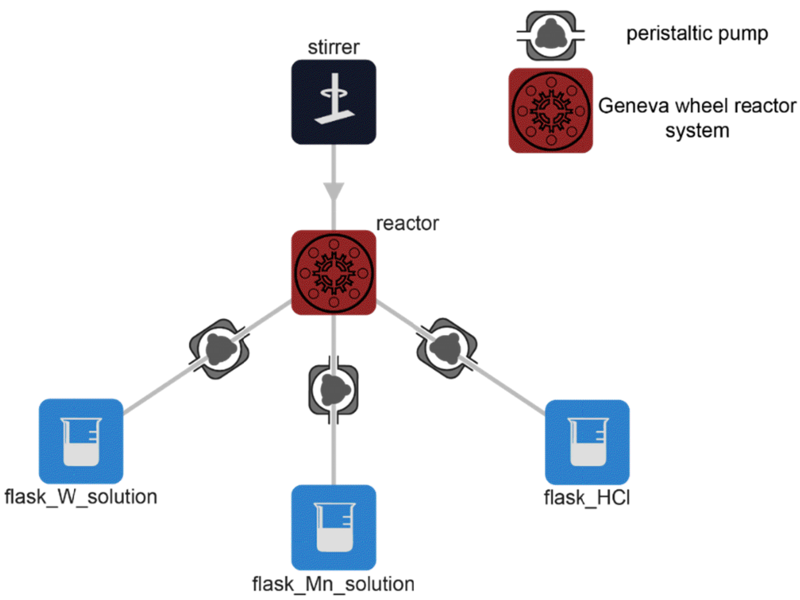


***Figure 11***

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2006243 A

### Non-patent literature cited in the description


- **BOECKMAN, R. K**. The Dess-Martin Periodinane: 1,1,1-Triacetoxy-1,1-Dihydro-1,2-Benziodoxol-3(1h)-One. *Organic Synthesis*, 2000, vol. 77, 141
- **FRIGERIO, M. et al.** A User-Friendly Entry to 2-Iodoxybenzoic Acid (IBX). *The Journal of Organic Chemistry*, 1999, vol. 64, 4537-4538
- **GOLDBERG, N. W. et al.** AlkylFluor: Deoxyfluorination of Alcohols. *Organic Letters*, 2016, vol. 18, 6102-6104
- **HAWIZY, L. et al.** ChemicalTagger: A tool for semantic text-mining in chemistry. *Journal of Cheminformatics*, 2011, vol. 3, 1-13
- **MENDOZA-ESPINOSA, D. et al.** Synthesis of 4- and 4,5-Functionalized Imidazol-2-ylidenes from a Single 4,5-Unsubstituted Imidazol-2-ylidene. *Journal of the America Chemical Society*, 2010, vol. 132, 7264-7265
- **NEUMANN, C. N et al.** Concerted nucleophilic aromatic substitution with 19F- and 18F. *Nature*, 2016, vol. 534, 369-373
- **REED, N. A. et al.** Circular Dichroism Investigation of Dess-Martin Periodinane Oxidation in the Organic Chemistry Laboratory. *Journal of Chemical Education*, 2005, vol. 82, 1053-1054
- **REILLY, T. J.** The Preparation of Lidocaine. *Journal of Chemical Education*, 1999, vol. 76, 1557
- **SLAUGHTER, L. A et al.** Pharmacological Treatment of Neonatal Seizures:A Systematic Review. *Journal of Child Neurology*, 2013, vol. 28, 351-364
- **STEINER, S. et al.** Organic synthesis in a modular robotic system driven by a chemical programming language. *Science*, 2019, vol. 363, 1-8
- **SYMES, M. D. et al.** Integrated 3D-printed reactionware for chemical synthesis and analysis. *Nature Chemistry*, 2012, vol. 4, 349-354
- **TANG, P. et al.** Deoxyfluorination of Phenols. *Journal of the America Chemical Society*, 2011, vol. 133, 11482-11484
- **TOJO, G** ; **FERNÁNDEZ, M**. Oxidation of alcohols to aldehydes and ketones : a guide to current common practice. Springer, 2010